# EUROPEAN PATENT APPLICATION

(11) **EP 4 585 684 A1**
(43) Date of publication of application: **16.07.2025**
(21) Application number: 23863258.2
(22) Date of filing: 07.09.2023
(51) Int. Cl.: C12N 15/11, A61K 31/713, A61K 47/54, A61K 48/00, A61P 35/00, C07H 21/02, C07H 21/04, C12N 15/113, C12N 15/87

(54) **NUCLEIC ACID FOR TRANSFECTION**

(30) Priority: 07.09.2022 JP 2022142206; 06.03.2023 JP 2023033984; 19.07.2023 JP 2023117905; 04.08.2023 JP 2023128179
(71) Applicant: AGC INC., Chiyoda-ku, Tokyo 1008405 (JP); The University of Tokyo, Bunkyo-ku, Tokyo 113-8654 (JP)
(72) Inventor: OKAZOE Takashi, Tokyo 100-8405 (JP); OKAMOTO Akimitsu, Tokyo 113-8654 (JP); MORIHIRO Kunihiko, Tokyo 113-8654 (JP); KOHATA Ai, Tokyo 113-8654 (JP)
(74) Representative: Müller-Boré & Partner Patentanwälte PartG mbB
(86) International application number: PCT/JP2023/032763
(87) International publication number: WO 2024/053720

(57) **Abstract**

The present invention provides a nucleic acid for transfection having excellent cell membrane permeability, and a method for producing the nucleic acid. The present invention relates to a nucleic acid for transfection, in which a nucleic acid targeted for introduction into a cell and a cell membrane-permeable group are linked together, and the cell membrane-permeable group has a structure represented by one of general formulas (A1) to (A4) (wherein in the formulas: R⁰ represents an alkyl group of 1 to 30 carbon atoms which may have an ether-bonded oxygen atom between carbon atoms and may be substituted with one or more fluorine atoms; n11, n12, n13 and n14 each independently represent an integer of 1 or greater; B represents a nucleic acid base; R^{FE} represents a perfluoroalkyl group of 1 to 10 carbon atoms which may have an ether-bonded oxygen atom between carbon atoms; na represents an integer of 1 to 10; and the black dots indicate bonding sites).

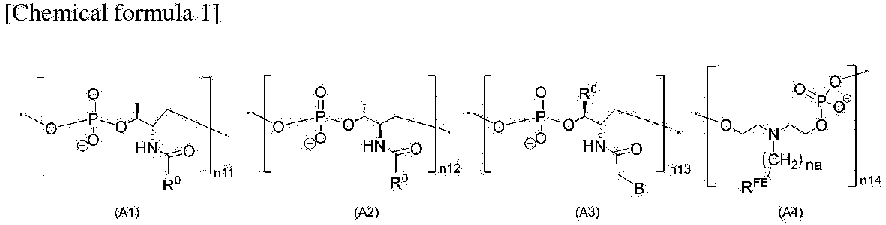

## Description

### [Technical Field]

The present invention relates to a nucleic acid for transfection having excellent cell membrane permeability, and a method for producing the same.

Priority is claimed on Japanese Patent Application No. 2022-142206, filed September 7, 2022, Japanese Patent Application No. 2023-033984, filed March 6, 2023, Japanese Patent Application No. 2023-117905, filed July 19, 2023, and Japanese Patent Application No. 2023-128179, filed August 4, 2023, the contents of which are incorporated herein by reference.

### [Background Art]

In recent years, research into nucleic acid drugs that use oligonucleotides continues to progress. Nucleic acid drugs have the excellent properties of exhibiting high specificity for the target molecule and having few side effects. However, in nucleic acids, the phosphodiester linkages are susceptible to nuclease degradation, and therefore nucleic acid drugs have stability problems when administered to living organisms. If the stability is low, then the nucleic acid may decompose in vivo before reaching the target tissue, meaning the desired drug efficacy may be unattainable. In order to improve nucleic acid stability, chimera nucleic acids formed with artificial nucleic acids having superior nuclease resistance and the like to natural nucleic acids are sometimes used. Examples of these artificial nucleic acids include acyclic glycol nucleic acids (GNA), peptide nucleic acids (PNA), acyclic threoninol nucleic acids (aTNA), and serinol nucleic acids (SNA) (Non-Patent Document 1).

Furthermore, nucleic acid drugs have poor cell membrane permeability, and delivering the drug to a target molecule located inside a cell is difficult. In particular, siRNA is double-stranded, and therefore has a higher molecular weight and a larger negative charge than antisense RNA, and inferior cell membrane permeability to antisense RNA, meaning a carrier is required for drug delivery. Known drug delivery agents include those that use lipid nanoparticles (Patent Document 1) and those that use cationic polymer nanoparticles (Patent Document 2). However, there remain many areas for improvement in terms of the efficiency of cell membrane permeability and toxicity concerns.

On the other hand, compounds having a polyfluoro structure are known to be stable and exhibit little toxicity in vivo, while offering excellent cellular uptake and endosomal escape (Non-Patent Document 2). Investigations are being conducted into utilizing these characteristics to introduce a polyfluoro structure as a portion having favorable cell membrane permeability into oligonucleotides and peptide nucleic acids (Patent Documents 3 and 4, Non-Patent Documents 3 to 6).

### [Citation List]

### [Patent Documents]

[Patent Document 1]
   International Patent Publication No. 2011/036557
[Patent Document 2]
   International Patent Publication No. 2017/212006
[Patent Document 3]
   International Patent Publication No. 2012/130941
[Patent Document 4]
   International Patent Publication No. 2021/060506
[Patent Document 5]
   Japanese Unexamined Patent Application, First Publication No. 2006-321797
[Patent Document 6]
   International Patent Publication No. 2000/056694

### [Non Patent Documents]

[Non Patent Document 1]
   Murayama et al., Chemistry A European Journal, 2013, vol. 19, pp. 14151 to 14158.
[Non Patent Document 2]
   Zhang et al., MRS Communications, 2018, vol. 8, pp. 303 to 313.
[Non Patent Document 3]
   Godeau et al., Medicinal Chemistry Communications, 2010, vol. 1. pp.76 to 78.
[Non Patent Document 4]
   Ellipilli et al., Chemical Communications, 2016, vol. 52, pp. 521 to 524.
[Non Patent Document 5]
   Rochambeaua et al., Polymer Chemistry, 2016, vol. 7, pp. 4998 to 5003.
[Non Patent Document 6]
   Metelev et al., Theranostics, 2017, vol. 7, pp. 3354 to 3368.
[Non Patent Document 7]
   Crombez, et al., Nucleic Acids Research, 2009, vol. 37(14), pp.4559 to 4569.

### [Summary of Invention]

### [Technical Problem]

The nucleic acid with a bonded cell membrane-permeable group disclosed in Non-Patent Document 3 has a problem in that it cannot easily be synthesized by the phosphoramidite method.

Non-Patent Document 4 describes a peptide nucleic acid imparted with superior cell membrane permeability by incorporating a polyfluoro structure, but makes no mention of nucleic acids.

Non-Patent Document 5 discloses a nucleic acid with an added polyfluoro structure, but makes no mention of cell membrane permeability, and uses a separate transfection reagent for intracellular introduction.

Non-Patent Document 6 discloses a nucleic acid with an added polyfluoro structure, but further improvements in the cell membrane permeability would be desirable.

The present invention has an object of providing a nucleic acid for transfection that exhibits excellent cell membrane permeability, and a method for producing such a nucleic acid.

### [Solution to Problem]

The inventors of the present invention discovered that by using, as a cell membrane-permeable group, a group obtained by introducing an alkyl group that may be substituted with fluorine atoms onto a side chain of an acyclic threoninol nucleic acid (aTNA nucleic acid), or a group in which the nucleoside portion of a phosphoramidite has been substituted with an organic group containing an ether bond-containing perfluoroalkyl group, and linking that cell membrane-permeable group to an siRNA targeted for introduction into a cell, the cell membrane permeability of the siRNA could be improved, and the siRNA could be introduced into a cell even without using a transfection reagent, thus enabling them to complete the present invention.

In other words, the present invention includes the following aspects.
[1] A nucleic acid for transfection in which a nucleic acid targeted for introduction into a cell and a cell membrane-permeable group are linked together, and
   the cell membrane-permeable group has a structure represented by one of general formulas (A1) to (A4) shown below: 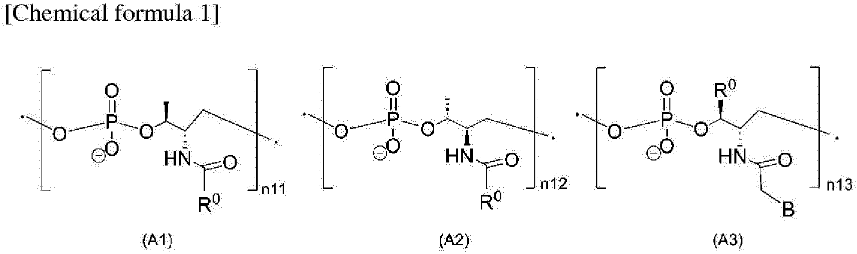 (wherein in formulas (A1) to (A3), R⁰ represents an alkyl group of 1 to 30 carbon atoms substituted with one or more fluorine atoms, a group composed of an alkyl group of 2 to 30 carbon atoms substituted with one or more fluorine atoms and having one to five ether-bonded oxygen atoms between the carbon atoms, an alkyl group of 10 to 30 carbon atoms not substituted with any fluorine atoms, or a group composed of an alkyl group of 10 to 30 carbon atoms not substituted with any fluorine atoms but having one to five ether-bonded oxygen atoms between the carbon atoms; n11, n12 and n13 each independently represent an integer of 1 or greater; B represents a nucleic acid base; and the black dots indicate bonding sites) 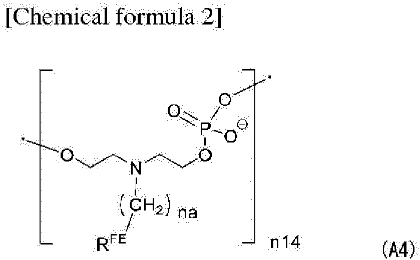 (wherein in formula (A4), R^{FE} represents a perfluoroalkyl group of 1 to 10 carbon atoms having no ether-bonded oxygen atoms between the carbon atoms, or a perfluoroalkyl group of 2 to 10 carbon atoms having one to five ether-bonded oxygen atoms between the carbon atoms; na represents an integer of 1 to 10; n14 represents an integer of 1 or greater; and the black dots represent bonding sites).
[2] The nucleic acid for transfection according to [1], wherein the nucleic acid targeted for introduction into a cell is an siRNA.
[3] The nucleic acid for transfection according to [1] or [2], wherein R⁰ represents an alkyl group of 1 to 30 carbon atoms substituted with at least two fluorine atoms.
[4] The nucleic acid for transfection according to [1] or [2], wherein R⁰ represents a perfluoroalkyl group of 1 to 10 carbon atoms, or a group composed of a perfluoroalkyl group of 1 to 10 carbon atoms having one to five ether-bonded oxygen atoms between the carbon atoms.
[5] The nucleic acid for transfection according to [1] or [2], wherein R⁰ represents an alkyl group of 10 to 30 carbon atoms not substituted with any fluorine atoms, or a group composed of an alkyl group of 10 to 30 carbon atoms not substituted with any fluorine atoms but having one to five ether-bonded oxygen atoms between the carbon atoms.
[6] The nucleic acid for transfection according to any one of [1] to [5], wherein n11 is 2 or greater, n12 is 2 or greater, n13 is 2 or greater, and n14 is 2 or greater.
[7] A nucleic acid transfection method, the method involving bringing the nucleic acid for transfection according to any one of [1] to [6] into contact with a cell to introduce the nucleic acid into the cell.
[8] A method for producing a cell membrane permeability-improved siRNA having a cell membrane-permeable group linked to an siRNA, wherein
   the cell membrane-permeable group has a structure represented by general formula (A1), (A2) or (A3) shown below: 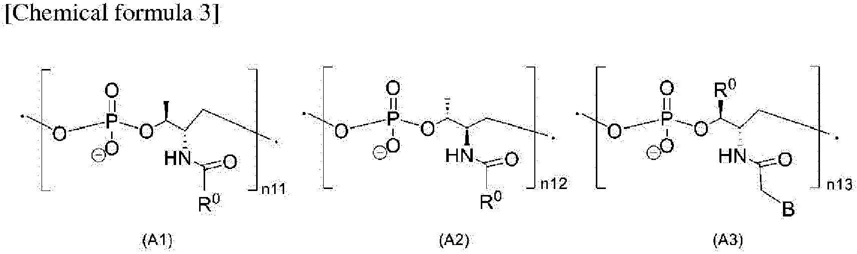 (wherein in formulas (A1) to (A3), R⁰ represents an alkyl group of 1 to 30 carbon atoms substituted with one or more fluorine atoms, a group composed of an alkyl group of 2 to 30 carbon atoms substituted with one or more fluorine atoms and having one to five ether-bonded oxygen atoms between the carbon atoms, an alkyl group of 10 to 30 carbon atoms not substituted with any fluorine atoms, or a group composed of an alkyl group of 10 to 30 carbon atoms not substituted with any fluorine atoms but having one to five ether-bonded oxygen atoms between the carbon atoms; n11, n12 and n13 each independently represent an integer of 1 or greater; B represents a nucleic acid base; and the black dots indicate bonding sites), and
   the method includes a step of linking the cell membrane-permeable group either directly or indirectly to the sense strand of the siRNA by the phosphoramidite method using a phosphoramidite having the cell membrane-permeable group as a raw material.
[9] An anticancer agent containing, as an active ingredient, a molecule in which an siRNA that targets a cancer gene and a cell membrane-permeable group are linked together either directly or indirectly, and
   the cell membrane-permeable group has a structure represented by one of general formulas (A1) to (A4) shown below: 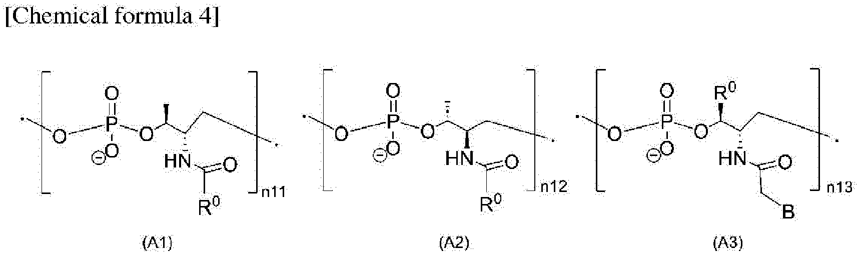 (wherein in formulas (A1) to (A3), R⁰ represents an alkyl group of 1 to 30 carbon atoms substituted with one or more fluorine atoms, a group composed of an alkyl group of 2 to 30 carbon atoms substituted with one or more fluorine atoms and having one to five ether-bonded oxygen atoms between the carbon atoms, an alkyl group of 10 to 30 carbon atoms not substituted with any fluorine atoms, or a group composed of an alkyl group of 10 to 30 carbon atoms not substituted with any fluorine atoms but having one to five ether-bonded oxygen atoms between the carbon atoms; n11, n12 and n13 each independently represent an integer of 1 or greater; B represents a nucleic acid base; and the black dots indicate bonding sites) 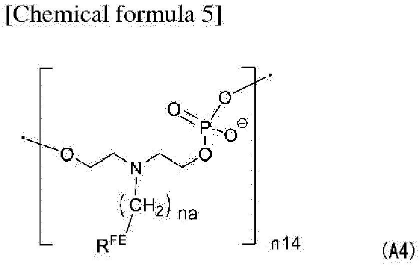 (wherein in formula (A4), R^{FE} represents a perfluoroalkyl group of 1 to 10 carbon atoms having no ether-bonded oxygen atoms between the carbon atoms, or a perfluoroalkyl group of 2 to 10 carbon atoms having one to five ether-bonded oxygen atoms between the carbon atoms; na represents an integer of 1 to 10; n14 represents an integer of 1 or greater; and the black dots represent bonding sites).

### [Advantageous Effects of Invention]

The nucleic acid for transfection according to the present invention has a specific structure with excellent cell membrane permeability, and therefore exhibits superior cell membrane permeability, and can introduce even an siRNA into a cell without requiring the use of a separate transfection reagent.

By applying the nucleic acid for transfection according to the present invention to an siRNA used as the active ingredient of an anticancer agent, an anticancer agent with superior efficacy can be produced.

### [Brief Description of Drawings]

FIG. 1 is a diagram illustrating the fluorescent intensity distributions at different culturing times for cells into which a fluorinated nucleic acid modified with N[C₈-PFC] at the 5'-terminal (PFC-ssDNA-FAM) or a control nucleic acid (ssDNA-FAM) has been introduced in Example 1.
FIG. 2 is a diagram schematically illustrating the structures of a control nucleic acid (ssDNA-FAM) and a nucleic acid (AM-ssDNA-FAM) having a phosphoramidite synthesized in a synthesis example linked to the 5'-terminal of this control nucleic acid, both of which were synthesized in Example 6.
FIG. 3 is a diagram illustrating the relative fluorescent intensity of cells into which an AM-ssDNA-FAM or a control nucleic acid (ssDNA-FAM) has been introduced in Example 6.
FIG. 4 is a diagram illustrating the relative fluorescent intensity of cells into which an AM-ssDNA-FAM or a control nucleic acid (ssDNA-FAM) has been introduced at either 4°C or 37°C in Example 6.
FIG. 5 is a diagram illustrating the relative fluorescent intensity of cells into which an siRNA (A2-ssRNA-FAM) containing D-aTNA-N4[FC10]-Luc sense-FAM or an siRNA (ssRNA-FAM) containing Luc sense-FAM has been introduced at 37°C in Example 11.
FIG. 6 is a diagram illustrating the measurement results for relative luciferase expression of cells into which various siRNAs have been introduced in Example 11.
FIG. 7 is a diagram illustrating the measurement results for relative luciferase expression of cells into which various siRNAs have been introduced in the presence of chloroquine in Example 11.
FIG. 8 is a diagram illustrating the relative fluorescent intensity of cells into which a fluorinated nucleic acid (D-aTNA-N2[FC10]) has been introduced in the presence of various different inhibitors in Example 12.

### [Description of Embodiments]

In the present invention and in this description, the term "nucleic acid" means a molecule in which nucleotides are bonded via phosphate diester bonds. These nucleotides include not only natural nucleotides (naturally occurring nucleotides) such as DNA and RNA, but also artificial nucleotides prepared by modifying a natural nucleotide to form a nucleotide capable of phosphate diester bonding with a natural nucleotide. Examples of artificial nucleotides include nucleotides in which a side chain or the like of a natural nucleotide has been modified with a functional group such as an amino group, nucleotides in which the hydroxyl group at the 2' position of a ribose skeleton has been substituted with a methoxy group, fluoro group, or methoxyethyl group or the like, phosphorothioate nucleotides (nucleotides in which the oxygen atom of a phosphate group has been substituted with a sulfur atom), morpholino nucleotides (nucleotides in which a ribose or deoxyribose has been substituted with a morpholine ring), BNA (Bridged Nucleic Acid), HNA (Hexitol Nucleic Acid), LNA (Locked Nucleic Acid), PNA (Peptide Nucleic Acid), TNA (Threose Nucleic Acid), GNA (Glycerol Nucleic Acid), and CeNA (Cyclohexenyl Nucleic Acid) and the like. Furthermore, the term "nucleic acid" also includes molecules in which only one or more natural nucleotides such as DNA or RNA are bonded via phosphate diester bonds, molecules in which one or more natural nucleotides and one or more artificial nucleotides are bonded via phosphate diester bonds, and only one or more artificial nucleotides are bonded via phosphate diester bonds.

In the present invention and in this description, "Cₚ₁₋ₚ₂" (wherein p1 and p2 are positive integers that satisfy p1<p2) means a group having p1 to p2 carbon atoms.

In the present invention and in this description, a "C₁₋₃₀ alkyl group" is an alkyl group having 1 to 30 carbon atoms, and may have either a linear chain or a branched chain. Similarly, a "C₂₋₃₀ alkyl group" is an alkyl group having 2 to 30 carbon atoms, and may have either a linear chain or a branched chain. Examples of the C₁₋₃₀ alkyl group include a methyl group, ethyl group, propyl group, isopropyl group, butyl group, isobutyl group, sec-butyl group, tert-butyl group, pentyl group, isopentyl group, neopentyl group, tert-pentyl group, hexyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, eicosyl group, heneicosyl group, docosyl group, tricosyl group, tetracosyl group, pentacosyl group, hexacosyl group, heptacosyl group, octacosyl group, nonacosyl group, and triacontyl group.

In the present invention and in this description, a "C₁₋₂₀ alkyl group" is an alkyl group having 1 to 20 carbon atoms, and may have either a linear chain or a branched chain. Similarly, a "C₂₋₂₀ alkyl group" is an alkyl group having 2 to 20 carbon atoms, and may have either a linear chain or a branched chain. Examples of the C₁₋₂₀ alkyl group include a methyl group, ethyl group, propyl group, isopropyl group, butyl group, isobutyl group, sec-butyl group, tert-butyl group, pentyl group, isopentyl group, neopentyl group, tert-pentyl group, hexyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, and eicosyl group.

In the present invention and in this description, a "C₁₋₁₀ alkyl group" is an alkyl group having 1 to 10 carbon atoms, and may have either a linear chain or a branched chain. Similarly, a "C₂₋₁₀ alkyl group" is an alkyl group having 2 to 10 carbon atoms, and may have either a linear chain or a branched chain. Examples of the C₁₋₁₀ alkyl group include a methyl group, ethyl group, propyl group, isopropyl group, butyl group, isobutyl group, sec-butyl group, tert-butyl group, pentyl group, isopentyl group, neopentyl group, tert-pentyl group, hexyl group, heptyl group, octyl group, nonyl group, and decyl group.

In the present invention and in this description, a "C₁₀₋₃₀ alkyl group" is an alkyl group having 10 to 30 carbon atoms, and may have either a linear chain or a branched chain. Examples of the C₁₀₋₃₀ alkyl group include an undecyl group, dodecyl group, tridecyl group, tetradecyl group, pentadecyl group, hexadecyl group, heptadecyl group, octadecyl group, nonadecyl group, eicosyl group, heneicosyl group, docosyl group, tricosyl group, tetracosyl group, pentacosyl group, hexacosyl group, heptacosyl group, octacosyl group, nonacosyl group, and triacontyl group.

In the present invention and in this description, a "C₁₋₆ alkyl group" is an alkyl group having 1 to 6 carbon atoms, and may have either a linear chain or a branched chain. Examples of the C₁₋₆ alkyl group include a methyl group, ethyl group, propyl group, isopropyl group, butyl group, isobutyl group, sec-butyl group, tert-butyl group, pentyl group, isopentyl group, neopentyl group, tert-pentyl group, and hexyl group.

In the present invention and in this description, an "alkylene group" is a divalent group in which two hydrogen atoms have been removed from a saturated hydrocarbon, and may have either a linear chain or a branched chain. Examples of the alkylene group include a methylene group, ethylene group, trimethylene group, tetramethylene group, pentamethylene group, hexamethylene group, heptamethylene group, octamethylene group, nonamethylene group, methylmethylene group, ethylmethylene group, methylethylene group, methylpropylene group, ethylethylene group, dimethylmethylene group, 1,2-dimethylethylene group, 1,1-dimethylethylene group, 1-ethylpropylene group, 2-ethylpropylene group, 1,2-dimethylpropylene group, 2,2-dimethylpropylene group, 1-propylpropylene group, 2-propylpropylene group, 1-methyl-1-ethylpropylene group, 1-methyl-2-ethylpropylene group, 1-ethyl-2-methylpropylene group, 2-methyl-2-ethylpropylene group, 1-methylbutylene group, 2-methylbutylene group, 3-methylbutylene group, 2-ethylbutylene group, 1-methylpentylene group, 2-ethylpentylene group, and 1-methylhexylene group.

In the present invention and in this description, a "C₁₋₂₀ perfluoroalkyl group" is a group in which all of the hydrogen atoms of an alkyl group having 1 to 20 carbon atoms have each been substituted with a fluorine atom. Examples of the C₁₋₂₀ perfluoroalkyl group include a perfluoromethyl group, perfluoroethyl group, perfluoropropyl group, perfluoroisopropyl group, perfluorobutyl group, perfluoroisobutyl group, perfluoro-sec-butyl group, perfluoro-tert-butyl group, perfluoropentyl group, perfluoroisopentyl group, perfluoroneopentyl group, perfluoro-tert-pentyl group, perfluorohexyl group, perfluoroheptyl group, perfluorooctyl group, perfluorononyl group, perfluorodecyl group, perfluoroundecyl group, perfluorododecyl group, perfluorotridecyl group, perfluorotetradecyl group, perfluoropentadecyl group, perfluorohexadecyl group, perfluoroheptadecyl group, perfluorooctadecyl group, perfluorononadecyl group, and perfluoroeicosyl group.

In the present invention and in this description, a "perfluoroalkylene group" is a group in which all of the hydrogen atoms of an alkylene group have each been substituted with a fluorine atom. Examples of the perfluoroalkylene group include groups in which all of the hydrogen atoms of the various alkylene groups listed above have each been substituted with a fluorine atom.

In the present invention and in this description, an "ether-bonded oxygen atom" describes an oxygen atom linking two carbon atoms, and does not include oxygen atoms that are linked in series to another oxygen atom. The number of ether-bonded oxygen atoms that an alkyl group of Nc carbon atoms (wherein Nc is an integer of 2 or greater) may have is a maximum of Nc-1. Further, a "C₂₋₁₀ alkyl group having an ether-bonded oxygen atom between carbon atoms" is a group having at least one ether-bonded oxygen atom between carbon atoms of the C₂₋₁₀ alkyl group. In the following description, an "alkyl group having an ether-bonded oxygen atom between carbon atoms" is sometimes referred to as an "ether bond-containing alkyl group".

In the present invention and in this description, a "C₂₋₁₀ perfluoroalkyl group having an ether-bonded oxygen atom between carbon atoms" is a group in which all of the hydrogen atoms of an ether bond-containing C₂₋₁₀ alkyl group (a group having at least one ether-bonded oxygen atom between carbon atoms of the C₂₋₁₀ alkyl group) have each been substituted with a fluorine atom. In the following description, this type of "perfluoroalkyl group having an ether-bonded oxygen atom between carbon atoms" is sometimes referred to as an "ether bond-containing perfluoroalkyl group".

In the present invention and in this description, a "halogen atom" means a fluorine atom, chlorine atom, bromine atom, or iodine atom. The expression "halogen atom other than a fluorine atom" means a chlorine atom, bromine atom, or iodine atom. This "halogen atom other than a fluorine atom" is preferably a chlorine atom or bromine atom, and a chlorine atom is particularly desirable.

Furthermore, in the following description, the expression "compound (n)" means a compound represented by formula (n).

The nucleic acid for transfection according to the present invention is a nucleic acid in which the nucleic acid targeted for introduction into a cell (the introduction target nucleic acid) and a cell membrane-permeable group are linked together. The introduction target nucleic acid typically has poor cell membrane permeability, but linking the cell membrane-permeable group improves the cell membrane permeability dramatically. Accordingly, the nucleic acid for transfection according to the present invention can be introduced into the cell interior simply by bringing the nucleic acid into contact with the cell surface, even without using a separate transfection reagent.

In the present invention and in this description, a "cell membrane-permeable group" means a group which, when added to a nucleic acid, can improve the cell membrane permeability of that nucleic acid. Specifically, the cell membrane-permeable group has a structure represented by one of general formulas (A1) to (A4) shown below. In the following description, a "structure represented by general formula (A1)" is sometimes referred to as a "structure (A1)", a "structure represented by general formula (A2)" is sometimes referred to as a "structure (A2)", a "structure represented by general formula (A3)" is sometimes referred to as a "structure (A3)", and a "structure represented by general formula (A4)" is sometimes referred to as a "structure (A4)".

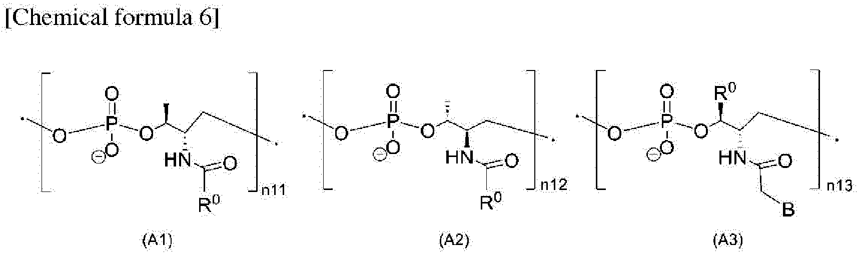

In general formulas (A1) to (A3), R⁰ represents a C₁₋₃₀ alkyl group substituted with at least one fluorine atom (a C₁₋₃₀ fluoroalkyl group), or a C₁₀₋₃₀ alkyl group not substituted with any fluorine atoms. In those cases where the alkyl group has two or more carbon atoms, the alkyl group may also have one to five ether -bonded oxygen atoms between the carbon atoms. In the present invention and in this description, the expression "C₁₋₃₀ fluoroalkyl group (wherein when the alkyl group has two or more carbon atoms, the alkyl group may also have one to five ether -bonded oxygen atoms between the carbon atoms)" means "a C₁₋₃₀ fluoroalkyl group, or a group composed of a C₂₋₃₀ fluoroalkyl group having one to five ether -bonded oxygen atoms between the carbon atoms".

In those cases where R⁰ represents a C₁₋₃₀ fluoroalkyl group, one or more hydrogen atoms bonded to the carbon atoms may also be substituted with a halogen atom other than a fluorine atom. In the structure (A1), the structure (A2) or the structure (A3), R⁰ is preferably a C₁₋₃₀ fluoroalkyl group substituted with at least two fluorine atoms. Among the various possibilities, R⁰ is preferably a C₁₋₂₀ fluoroalkyl group, more preferably a C₁₋₁₅ fluoroalkyl group, even more preferably a C₂₋₁₅ fluoroalkyl group, and still more preferably a C₆₋₁₀ fluoroalkyl group. In those cases where R⁰ is a C₁₋₃₀ fluoroalkyl group, there are no particular limitations on the number of hydrogen atoms substituted with a fluorine atom, provided one or more hydrogen atoms are substituted with a fluorine atom, but for example, the number of fluorine-substituted hydrogen atoms is preferably three or more, more preferably six or more, and even more preferably seven or more.

In those cases where R⁰ represents a C₁₋₃₀ fluoroalkyl group, groups represented by general formula (f-1) or (f-2) shown below are preferred as R⁰. In these formulas, Rf^{P} represents a fully halogenated C₁₋₂₀ alkyl group (a group in which all of the hydrogen atoms of a C₁₋₂₀ alkyl group have each been substituted with a halogen atom) having one or more fluorine atoms. In those cases where Rf^{P} has two or more carbon atoms, namely in the case of a fully halogenated C₂₋₂₀ alkyl group, the group may also have one to five ether-bonded oxygen atoms between the carbon atoms. In the present invention and in this description, the expression "fully halogenated C₂₋₂₀ alkyl group that may have one to five ether-bonded oxygen atoms between the carbon atoms" means "a fully halogenated C₂₋₂₀ alkyl group, or a group composed of a fully halogenated C₂₋₂₀ alkyl group having one to five ether-bonded oxygen atoms between the carbon atoms".

In general formula (f-2), the two Rf^{P} groups may represent the same type of group or different groups. Rf^{P} is preferably a C₁₋₂₀ perfluoroalkyl group (a group composed of a C₁₋₂₀ alkyl group in which all of the hydrogen atoms have each been substituted with a fluorine atom).

In general formula (f-1) or (f-2) shown below, n1 represents an integer of 0 to 10, and n2 represents an integer of 0 to 9. When n1 and n2 are 0, they each represent a single bond. In other words, when n1 is 0, the group represented by general formula (f-1) is simply -Rf^{p}, and when n2 is 0, the group represented by general formula (f-2) is - CH(Rf^{p})₂.

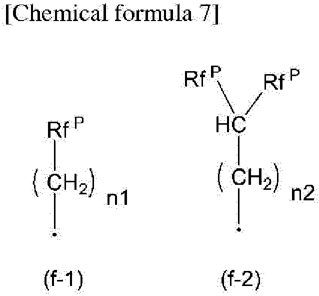

In those cases where R⁰ is a group represented by general formula (f-1), R⁰ is preferably a group in which Rf^{P} represents a trifluoromethyl group, pentafluoroethyl group, heptafluoropropyl group, nonafluorobutyl group, perfluoropentyl group, perfluorohexyl group, perfluoroheptyl group, perfluorooctyl group, perfluorononyl group or perfluorodecyl group, and n1 is an integer of 0 to 4, more preferably a group in which Rf^{P} represents a trifluoromethyl group, pentafluoroethyl group, heptafluoropropyl group, nonafluorobutyl group, perfluoropentyl group, perfluorohexyl group, perfluoroheptyl group, perfluorooctyl group, perfluorononyl group or perfluorodecyl group, and n1 is an integer of 0 to 2, even more preferably a group in which Rf^{P} represents a trifluoromethyl group, pentafluoroethyl group, heptafluoropropyl group, nonafluorobutyl group, perfluoropentyl group or perfluorohexyl group, and n1 is an integer of 0 to 2 (but excluding the case where n1 is 1 and Rf^{P} is a trifluoromethyl group), and still more preferably a group in which Rf^{P} represents a pentafluoroethyl group, heptafluoropropyl group, nonafluorobutyl group, perfluoropentyl group, perfluorohexyl group, perfluoroheptyl group, perfluorooctyl group, perfluorononyl group or perfluorodecyl group, and n1 is 0.

In those cases where R⁰ is a group represented by general formula (f-2), R⁰ is preferably a group in which Rf^{P} represents a trifluoromethyl group, pentafluoroethyl group, heptafluoropropyl group, nonafluorobutyl group, perfluoropentyl group, perfluorohexyl group, perfluoroheptyl group, perfluorooctyl group, perfluorononyl group or perfluorodecyl group, and n2 is an integer of 0 to 4, more preferably a group in which Rf^{P} represents a trifluoromethyl group, pentafluoroethyl group, heptafluoropropyl group, nonafluorobutyl group, perfluoropentyl group, perfluorohexyl group, perfluoroheptyl group, perfluorooctyl group, perfluorononyl group or perfluorodecyl group, and n2 is an integer of 0 to 2, even more preferably a group in which Rf^{P} represents a trifluoromethyl group, pentafluoroethyl group, heptafluoropropyl group, nonafluorobutyl group, perfluoropentyl group or perfluorohexyl group, and n2 is an integer of 0 to 2 (but excluding the cases where n2 is 0 or 1 and Rf^{P} is a trifluoromethyl group), and still more preferably a group in which Rf^{P} represents a pentafluoroethyl group, heptafluoropropyl group, nonafluorobutyl group, perfluoropentyl group or perfluorohexyl group, and n2 represents 0.

In those cases where R⁰ represents a C₁₋₃₀ fluoroalkyl group, specific examples of R⁰ include a trifluoromethyl group, pentafluoroethyl group, heptafluoropropyl group, nonafluorobutyl group, perfluoropentyl group, perfluorohexyl group, perfluoroheptyl group, perfluorooctyl group, perfluorononyl group or perfluorodecyl group, difluoromethyl group, 1,1-difluoroethyl group, 2,2-difluoroethyl group, 1,1,2,2-tetrafluoroethyl group, 1,1,2,2,3,3-hexafluoropropyl group, 1,1,2,3,3,3-hexafluoropropyl group, 1,1,2,2,3,3-hexafluorohexyl group, 1,1,2,2,3,3-hexafluorooctyl group, 1,1,2,2,3,3-hexafluorodecyl group, 1,1,2,2,3,3-hexafluorooctadecyl group, and 1,1,2,2,3,3-hexafluorohexacosyl group. The nucleic acid for transfection according to the present invention is preferably a nucleic acid having a structure represented by general formula (A1), general formula (A2) or general formula (A3) in which R⁰ is a C₁₋₃₀ perfluoroalkyl group, is more preferably a nucleic acid having a structure represented by general formula (A1), general formula (A2) or general formula (A3) in which R⁰ is a C₁₋₂₀ perfluoroalkyl group, and is even more preferably a nucleic acid having a structure represented by general formula (A1), general formula (A2) or general formula (A3) in which R⁰ is a C₁₋₁₀ perfluoroalkyl group.

In those cases where R⁰ represents a C₁₀₋₃₀ alkyl group, the structure (A1), the structure (A2) or the structure (A3) is preferably a structure in which R⁰ is a C₁₀₋₂₅ alkyl group, more preferably a C₁₅₋₂₅ alkyl group, and even more preferably a C₁₅₋₂₃ alkyl group. Alkyl groups of sufficient length have high hydrophobicity similar to fluoroalkyl groups, and contribute to the cell membrane permeability of a nucleic acid having the structure (A1), the structure (A2) or the structure (A3).

In general formula (A3), B represents a nucleic acid base. This nucleic acid base is preferably a nucleic acid base of a natural nucleic acid, a nucleic acid base having a similar structure to a natural nucleic acid base, or a modified base in which one of these bases has been subjected to any of various modifications. Examples of possible base modifications include alkylation, hydroxylation, alkoxylation, acylation, dihydrogenation, amination, formylation, and halogenation. Examples of nucleic acid bases having a similar structure to a natural nucleic acid base include triazole, imidazole, azapyrimidine, and azapurine. Specific examples of the nucleic acid base for B include adenine, guanine, cytosine, thymine, uracil, 1-methyladenine, N6-methyladenine, 7-methylguanine, 5-methylcytosine, 1-methylthymine, 5-methyluracil, 5-hydroxymethylcytosine, 5-hydroxyuracil, 5-hydroxymethyluracil, dihydrouracil, dihydrothymine, dihydrocytosine, 2,6-diaminoadenine, 2,6-diaminoguanine, 6-thioguanine, 2-thioadenine, 2-thiocytosine, 4-thiouracil, 5-fluorouracil, 5-iodouracil, 5-halogenocytosine, 5-fluorocytosine, 5-trihalogenomethyluracil, 5-trifluoromethyluracil, 5-azathymine, 5-azacytosine, 6-azauracil, 8-azaadenine, 7-deazaadenine, 7-deazaguanine, and 3-deazauracil. The nucleic acid for transfection according to the present invention is preferably a nucleic acid having a structure in which B in general formula (A3) is adenine, guanine, cytosine, thymine, or uracil.

In general formulas (A1) to (A3), n11, n12 and n13 each independently represent an integer of 1 or greater. Further, n11, n12 and n13 represent the number of repeating units of each structure per molecule, and the higher this number, the more the hydrophobicity increases. As a result, the cell membrane permeability of the nucleic acid also improves. In the nucleic acid for transfection according to the present invention, n11, n12 and n13 are preferably each 2 or greater, and more preferably 5 or greater. Further, in terms of making it easier to adopt a structure similar to a natural double-stranded nucleic acid or single-stranded nucleic acid, n11, n12 and n13 are preferably each not more than 10, more preferably not more than 8, and even more preferably 6 or less. In those cases where n11, n12 and n13 are 2 or greater, the plurality of the structures (A1), structures (A2) or structures (A3) may be the same or different.

When a nucleic acid having an introduced structure (A1), structure (A2) or structure (A3) forms a DNA double helix structure, the R⁰ group is exposed on the outside of the helix structure, and therefore a stable double helix structure can be formed, and the R⁰ groups exposed at the surface improve the cell membrane permeability. In addition, a nucleic acid having an introduced structure (A1), structure (A2) or structure (A3) contains no nitrogen atoms within the chain formed from the phosphodiester linkages between the phosphate groups and the sugars of the nucleic acid, and therefore intermediates formed, for example, during synthesis by the phosphoramidite method are comparatively stable, making synthesis easier.

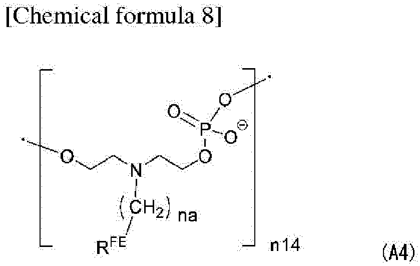

In general formula (A4), R^{FE} represents a C₁₋₁₀ perfluoroalkyl group having no ether-bonded oxygen atoms between the carbon atoms, or a C₂₋₁₀ perfluoroalkyl group having one to five ether-bonded oxygen atoms between the carbon atoms (an ether-bonded oxygen-containing C₂₋₁₀ perfluoroalkyl group). Further, in general formula (A4), na represents an integer of 1 to 10.

In those cases where R^{FE} is a C₁₋₁₀ perfluoroalkyl group having no ether-bonded oxygen atoms between the carbon atoms, the group may have either a linear chain or a branched chain. A C₁₋₁₀ perfluoroalkyl group provides satisfactory cell membrane permeability, and cytotoxicity is also suppressed. The structure (A4) in a nucleic acid for transfection according to the present invention is preferably a linear C₂₋₁₀ perfluoroalkyl group, more preferably a linear C₃₋₁₀ perfluoroalkyl group, and even more preferably a linear C₄₋₁₀ perfluoroalkyl group.

In those cases where R^{FF} is an ether-bonded oxygen-containing C₂₋₁₀ perfluoroalkyl group, a group represented by, for example, any of general formulas (F1) to (F3) shown below is preferred.
[Chemical formula 9]

-R^{F1}-O-R^{F2} (F1)

-R^{F3}-(O-CF₂-CF₂-)n1-O-R^{F4} (F2)

-R^{F5}-(O-CF₂-CF₂-CF₂-)n2-O-R^{F6} (F3)

In general formula (F1), R^{F1} represents a perfluoroalkylene group having q1 carbon atoms and R^{F2} represents a perfluoroalkyl group having q2 carbon atoms. Further, q1 and q2 are natural numbers, the sum of which is at least 2 but not more than 10.

In general formula (F2), R^{F3} represents a perfluoroalkylene group having q3 carbon atoms and R^{F4} represents a perfluoroalkyl group having q4 carbon atoms. Further, n1, q3 and q4 are natural numbers for which the value of 2×n1+q3+q4 is at least 4 but not more than 10.

In general formula (F3), R^{F5} represents a perfluoroalkylene group having q5 carbon atoms and R^{F6} represents a perfluoroalkyl group having q6 carbon atoms. Further, n2, q5 and q6 are natural numbers for which the value of 3×n2+q5+q6 is at least 5 but not more than 10.

R^{F1} in general formula (F1), R^{F3} in general formula (F2) and R^{F5} in general formula (F3) may each represent a linear perfluoroalkylene group or a branched perfluoroalkylene group. Each of R^{F1}, R^{F3} and R^{F5} is preferably a C₁₋₃ perfluoroalkylene group, more preferably a group in which all of the hydrogen atoms of a methylene group, ethylene group, methylmethylene group, ethylmethylene group, dimethylmethylene group or methylethylene group have each been substituted with a fluorine atom, even more preferably a group in which all of the hydrogen atoms of a methylene group, methylmethylene group, ethylmethylene group or dimethylmethylene group have each been substituted with a fluorine atom, and even more preferably a group in which all of the hydrogen atoms of a methylene group or a methylmethylene group have each been substituted with a fluorine atom.

R^{F2} in general formula (F1), R^{F4} in general formula (F2) and R^{F6} in general formula (F3) may each represent a linear perfluoroalkyl group or a branched perfluoroalkyl group. Each of R^{F2}, R^{F4} and R^{F6} is preferably a linear or branched C₁₋₅ perfluoroalkyl group, more preferably a linear C₁₋₅ perfluoroalkyl group, and even more preferably a linear C₂₋₄ perfluoroalkyl group.

In the present invention, the ether bond-containing perfluoroalkyl group introduced into the nucleic acid may, for example, use the ether bond-containing perfluoroalkyl group included in the fluorine compound disclosed in Patent Document 5 or 6 together with a production method therefor.

In general formula (A4), n14 represents an integer of 1 or greater. Further, n14 represents the number of repeating units of the structure (A4) per molecule, and the higher this number, the more the hydrophobicity increases, and the more the cell membrane permeability of the nucleic acid improves. In the nucleic acid for transfection according to the present invention, n14 is preferably 2 or greater, and more preferably 5 or greater. Further, in terms of making it easier to adopt a structure similar to a natural double-stranded nucleic acid or single-stranded nucleic acid, n14 is preferably not more than 10, more preferably not more than 8, and even more preferably 6 or less. In those cases n14 is 2 or greater, the plurality of the structures (A4) may be the same or different.

In those cases where the nucleic acid for transfection according to the present invention has two or more cell membrane-permeable groups per molecule, the plurality of cell membrane-permeable groups may be of the same type or different types. For example, the nucleic acid for transfection according to the present invention may have the structure (A1) and the structure (A2), may have the structure (A1) and the structure (A3), may have the structure (A1) and the structure (A4), may have the structure (A2) and the structure (A3), may have the structure (A2) and the structure (A4), may have the structure (A3) and the structure (A4), may have the structure (A1), the structure (A3) and the structure (A4), may have the structure (A1), the structure (A2) and the structure (A4), may have the structure (A1), the structure (A3) and the structure (A4), may have the structure (A1), the structure (A2) and the structure (A3), or may have the structure (A2), the structure (A3) and the structure (A4).

In general formulas (A1) to (A4), the black dots represent bonding sites. The nucleic acid for transfection according to the present invention may have any of the structures (A1) to (A4) and there are no particular limitations on the location at which each of the structures (A1) to (A4) is introduced, and each structure may be introduced at any location that does not impair the function of the nucleic acid. For example, the structures (A1) to (A4) may be bonded either directly or indirectly to the 5'-terminal or the 3'-terminal of the nucleic acid, or may be introduced between two nucleotides within the nucleic acid.

Among the various structures for the nucleic acid for transfection according to the present invention, in nucleic acids having the structure (A1) or the structure (A2) at the 5'-terminal of the nucleic acid, it is preferable that the bonding site extending from the carbon atom at one terminal of the structure (A1), the structure (A2) or the structure (A3) is bonded to a hydroxyl group, while the bonding site extending from the oxygen atom of the phosphate group at the other terminal of the structure (A1), the structure (A2) or the structure (A3) is bonded to the sugar at the 5'-terminal of the nucleic acid. Among the various structures for the nucleic acid for transfection according to the present invention, in nucleic acids having the structure (A1), the structure (A2) or the structure (A3) at the 3'-terminal of the nucleic acid, it is preferable that the bonding site extending from the carbon atom at one terminal of the structure (A1), the structure (A2) or the structure (A3) is bonded to the phosphate group at the 3'-terminal of the nucleic acid, while the bonding site extending from the oxygen atom of the phosphate group at the other terminal of the structure (A1), the structure (A2) or the structure (A3) is bonded to a hydrogen atom.

Among the various structures for the nucleic acid for transfection according to the present invention, in nucleic acids having the structure (A1), the structure (A2) or the structure (A3) introduced between two nucleotides, it is preferable that the bonding site extending from the carbon atom at one terminal of the structure (A1), the structure (A2) or the structure (A3) is bonded to the phosphate group of another nucleotide, while the bonding site extending from the oxygen atom of the phosphate group at the other terminal of the structure (A1), the structure (A2) or the structure (A3) is bonded to the sugar of another nucleotide, as such structures are more similar to the structures of natural nucleic acids.

In the nucleic acid for transfection according to the present invention, the cell membrane-permeable group and the introduction target nucleic acid may be linked directly, or may be linked indirectly via a linking group. There are no particular limitations on this linking group, and any group that enables linking of the introduction target nucleic acid and the cell membrane-permeable group may be used, including typical divalent or trivalent organic groups. Examples of the linking group include an alkylene group, alkenylene group, carbonyl group, amino group, ether linkage, thioether linkage, ester linkage, amide linkage, polyethylene glycol group (PEG: -(C₂H₄O)ₙ-), siloxane linkage, silyl ether linkage, a sugar, a peptide, and a nucleotide chain. Further, groups in which two or three hydrogen atoms have been removed from a ring such as a pyrrole ring, pyrazole ring, imidazole ring, triazole ring, pyridine ring, pyrimidine ring, pyrazine ring, oxazole ring, thiazole ring, furan ring, thiophene ring or benzene ring may also be used as the linking group. Moreover, appropriate combinations of the above groups may also be used.

In the nucleic acid for transfection according to the present invention, the structures (A1) to (A4) can be linked to the introduction target nucleic acid using any of various coupling reactions. For example, by performing the phosphoramidite method using a phosphoramidite containing the structure (A1) as a raw material, the structure (A1) can be easily introduced into the nucleic acid. Similarly, by performing the phosphoramidite method using a phosphoramidite containing the structure (A2), the structure (A3) or the structure (A4) as a raw material, the structure (A2), the structure (A3) or the structure (A4) can be easily introduced into the nucleic acid. Most widely used nucleic acid automatic synthesis devices utilize the phosphoramidite method. Accordingly, by using a phosphoramidite containing one of the structures (A1) to (A4) as a raw material, a nucleic acid having one of the structures (A1) to (A4) introduced at a desired location within a nucleic acid of any of various base sequences can be synthesized easily using an automatic synthesis device.

Examples of the phosphoramidite containing the structure (A1), the structure (A2) or the structure (A3) include compounds in which the sugar and the phosphate group of any of the phosphoramidites typically used in nucleic acid synthesis have been linked via the structure (A1), and compounds in which the nucleoside portion of any of the phosphoramidites typically used in nucleic acid synthesis has been substituted with an organic group containing the structure (A3).

Examples of the phosphoramidite containing the structure (A4) include compounds represented by general formula (A4-0) shown below. In general formula (A4-0), R^{FE} and na are the same as in general formula (A4). Further, i-Pr represents an isopropyl group, and DMTr represents a 4,4'-dimethoxytriphenylmethyl group.

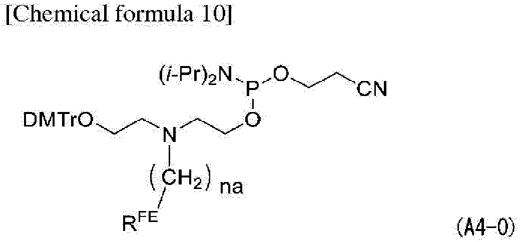

In addition, the cell membrane-permeable group can also be introduced into the introduction target nucleic acid using any of the methods disclosed in Non-Patent Documents 3 to 6.

The synthesized nucleic acid can be isolated and purified by any of various methods such as ion chromatography, gel permeation chromatography, reversed-phase chromatography, and normal phase chromatography.

There are no particular limitations on the introduction target nucleic acid within the nucleic acid for transfection according to the present invention, provided the nucleic acid requires introduction into a cell. A functional nucleic acid which exhibits some form of bioactivity when introduced into the target cell in vivo is preferred as the introduction target nucleic acid. Specific examples include nucleic acids for expressing a protein inside the cell, nucleic acids for suppressing gene expression inside the cell, nucleic acids for conducting genetic modification, nucleic acid aptamers that bind specifically to a target biomolecule, and functional nucleic acids that effect the physiological function of the cell. Examples of nucleic acids for protein expression include cDNA and mRNA that encode a protein, and the nucleic acid may be an expression plasmid vector incorporating a region that encodes the target protein. Examples of nucleic acids for suppressing gene expression include functional nucleic acids used in RNA interference such as siRNA, miRNA, shRNA, and antisense oligonucleotides, and RNAi vectors may also be used. Examples of nucleic acids for conducting genetic modification include genomic DNA fragments and the like. Examples of functional nucleic acids that effect the physiological function of the cell include decoy nucleic acids and CpG (cytosine-phosphate-guanine) oligonucleotides. In addition, nucleic acids having a functional molecule such as a fluorescent substance or a functional peptide or the like linked to the nucleic acid may also be used. For example, a nucleic acid molecule having a fluorescent substance linked to the terminal of a single-stranded nucleic acid that adopts a guanine quadruplex (G-quadruplex) may be used as the introduction target nucleic acid.

There are no particular limitations on the introduction target nucleic acid within the nucleic acid for transfection according to the present invention, and either a nucleic acid in which all of the included nucleotides are natural nucleotides or a nucleic acid in which some or all of the nucleotides are artificial nucleotides may be used. Further, the nucleic acid may be a single-stranded nucleic acid or a double-stranded nucleic acid. The nucleic acid may be a DNA, an RNA, or a chimeric nucleic acid of DNA and RNA.

In the nucleic acid for transfection according to the present invention, there are no particular limitations on the location at which the cell membrane-permeable group is introduced, and the group may be introduced at any location that does not impair the function of the nucleic acid. For example, the cell membrane-permeable group may be bonded to the 5'-terminal or the 3'-terminal of the introduction target nucleic acid, or may be introduced between two nucleotides within the introduction target nucleic acid.

The nucleic acid for transfection according to the present invention may be subjected to various modifications, provided that the cell membrane permeability provided by the perfluoroalkyl group is not impaired and the function of the introduction target nucleic acid is not impaired. Examples of these modifications include glycosylation, lipid modification, and peptide modification.

The nucleic acid for transfection according to the present invention contains a cell membrane-permeable group containing a perfluoroalkyl group, and therefore exhibits superior cell membrane permeability to a nucleic acid that does not contain such a group. Accordingly, the nucleic acid for transfection according to the present invention can be introduced into a cell simply by bring the nucleic acid into contact with the cell, without requiring the use of a typical transfection reagent such as lipofectamine. In those cases where the cell targeted for introduction of the nucleic acid for transfection according to the present invention is a cultured cell, the nucleic acid for transfection can be introduced into the cell simply by adding the nucleic acid for transfection to the culture medium and conducting cell culturing. Further, in the case of an animal tissue, the nucleic acid for transfection can be introduced into the cells constituting the tissue, for example, by spraying or coating a solution containing the dissolved nucleic acid for transfection according to the present invention onto the tissue surface. Accordingly, for example, by producing the nucleic acid for transfection according to the present invention using, as the introduction target nucleic acid, a functional nucleic acid that exhibits some form of bioactivity when introduced into the target cell in vivo, the uptake efficiency of that functional nucleic acid into the target cell can be improved. In other words, by using the nucleic acid for transfection according to the present invention, a drug delivery system that delivers a nucleic acid drug into a target cell can be constructed with ease.

The structures (A1) to (A4) can improve the cell membrane permeability without excessively damaging the double helix structure of the nucleic acid. Accordingly, a nucleic acid for transfection according to the present invention that uses an siRNA as the introduction target nucleic acid, namely a molecule having a cell membrane-permeable group formed from one of the structures (A1) to (A4) linked to an siRNA represents an siRNA in which the cell membrane permeability has been improved sufficiently to enable introduction of the nucleic acid into a target cell by transfection (namely, a cell membrane permeability-improved siRNA).

In this type of cell membrane permeability-improved siRNA, the siRNA and the cell membrane-permeable group formed from one of the structures (A1) to (A4) may be linked directly, or may be linked indirectly via a linking group. There are no particular limitations on the linking group, and any of the linking groups exemplified above may be used as appropriate. The cell membrane permeability-improved siRNA can be produced, for example, by the phosphoramidite method using a phosphoramidite having one of the structures (A1) to (A4) as a raw material.

Further, siRNAs that target cancer genes are used as the active ingredient of anticancer agents. Accordingly, a molecule having a cell membrane-permeable group formed from one of the structures (A1) to (A4) linked either directly or indirectly to an siRNA that targets a cancer gene exhibits excellent uptake efficiency into the targeted cancer cells, and is desirable as the active ingredient of an anticancer agent. There are no particular limitations on the cancer gene, and any gene that causes some form of abnormality such as causing cancer within normal cells may be targeted, with specific examples including the Cyclin B1 gene, SRC gene, Ras gene, and Myc gene. Known siRNA can be used as the siRNA for targeting these genes, and can be designed by normal methods from the various gene genome sequences.

For example, the Cyclin B1 gene has known relationships with esophageal squamous cell carcinoma, pharyngeal squamous cell carcinoma, colon cancer, head and neck carcinoma, non-small cell lung cancer, breast cancer, and cervical cancer and the like, and an siRNA that targets the Cyclin B1 gene can be used as the active ingredient of a pharmaceutical composition for treating or preventing these cancers. Accordingly, by linking a cell membrane-permeable group formed from one of the structures (A1) to (A4) either directly or indirectly to an siRNA that targets the Cyclin B1 gene, an anticancer agent that exhibits excellent efficacy against these types of cancers can be produced.

### [Examples]

The present invention is described below using a series of examples, but the present invention is not limited to these examples.

### <Cell Cultures>

In the experiments described below, cell culturing was conducted in the following manner.

HeLa cells (from the RIKEN cell bank) were cultured using a low-glucose Dulbecco's Modified Eagle Medium (DMEM, manufactured by FUJIFIL Wako Pure Chemical Corporation) to which 10% of FBS and 0.5% of a mixed solution of penicillin-streptomycin had been added as the culture medium, with culturing conducted under a humidified atmosphere (5% by volume CO₂) at a temperature of 37°C.

Twenty four hours prior to the introduction of the nucleic acid into the cells, the cells were inoculated into a 96-well culture plate (manufactured by Bioramo LLC) at a concentration of 1.0 × 10⁴ cells/well, and subjected to adhesion and proliferation (preculturing).

### <DNA Synthesis>

DNA syntheses were conducted using commercially available reagents, various phosphoramidites (acetonitrile solutions, 0.1 M), and 5-ethylthio-1H-tetrazole (acetonitrile solution, 0.25 M) as an activator, using an NTS H-8 DNA/RNA synthesizer (manufactured by Nihon Techno Service Co., Ltd.).

### [Synthesis Example 1]

A phosphoramidite containing a perfluoroalkyl group having 8 carbon atoms (C₈F₁₇-: hereinafter referred to as a C₈-PFC group) was synthesized, and using this phosphoramidite, a nucleic acid containing the C₈-PFC group was produced by the amidite method.

### <Synthesis of Phosphoramidite Containing C₈-PFC group>

The phosphoramidite containing the C₈-PFC group (N[Cs-PFC] amidite), represented by the formula shown below, was synthesized using the method disclosed in Non-Patent Document 5.

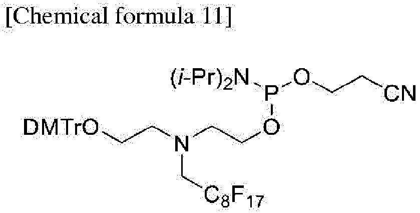

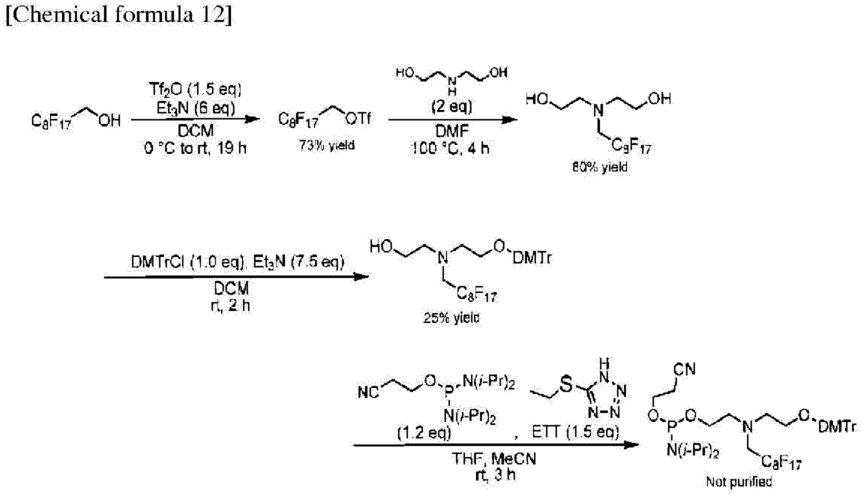

### [Synthesis Example 2]

A nucleic acid having the structure (A1) wherein R⁰ is a group represented by general formula (f-1) in which n1 is 2 and Rf^{P} represents a perfluorohexyl group was synthesized.

A phosphoramidite (D-aTNA-N[FC8]) having the structure (A1) wherein R⁰ is a group represented by general formula (f-1) in which n1 is 2 and Rf^{P} represents a perfluorohexyl group was synthesized as shown below.

### (1) Amidite Condensation

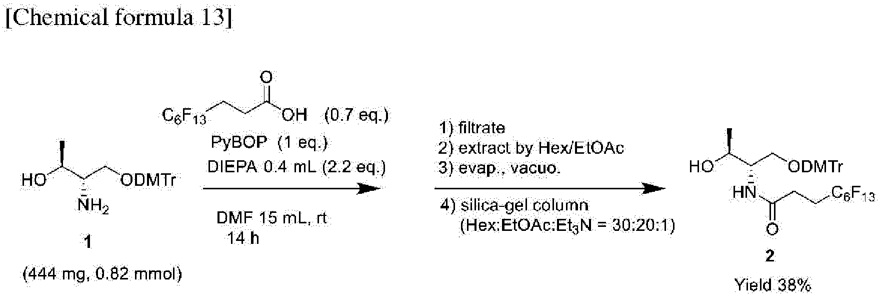

A compound 1 (0.44 g, 0.82 mmol) dissolved in 10 mL of DMF, PyBOP (0.43 g, 0.82 mmol) and DIPEA (0.4 mL, 2.2 equivalents) were dissolved in 15 mL of DMF under an argon atmosphere, and 4,4,5,5,6,6,7,7,8,8,9,9,9-tridecafluorononanoic acid (0.23 g, 0.59 mmol, 0.7 equivalents) was then added to complete preparation of the reaction solution. The thus obtained reaction solution was stirred at room temperature for 14 hours. Subsequently, the reaction mixture was quenched with water (45 mL) and then extracted twice with hexane / ethyl acetate (4:1 (volumetric ratio)). The organic fractions were combined, washed with water, dried over Na₂SO₄ and filtered, and the solvent was then removed under vacuum. The resulting crude product was purified by silica gel column chromatography (hexane : ethyl acetate : Et₃N = 30:20:1 (volumetric ratio)), yielding a compound 2 (0.17 g, 0.22 mmol, yield: 38%).

### Compound 2:

¹H NMR (400 MHz, CDCl₃) δ 7.39 to 7.21 (m, 9H), 6.84 (d, J = 5.9 Hz, 4H), 6.06 (d, J = 9.1 Hz, 1H), 4.13 to 4.10 (m, 1H), 3.92 to 3.90 (m, 1H), 3.78 (s, 6H), 3.41 (dd, J = 11.2, 5.7 Hz, 1H), 3.33 (dd, J = 9.6, 3.2 Hz, 1H), 2.90 (s, 1H), 2.53 to 2.44 (m, 4H), 1.12 (d, J = 6.4 Hz, 3H).

¹⁹F NMR (376 MHz, CDCl₃) δ -80.7 (s, 3F), -114.4 (s, 2F), -121.8 (s, 2F), -122.8 (s, 2F), -123.4 (s, 2F), -126.0 (s, 2F).

### (2) DMTr Protection

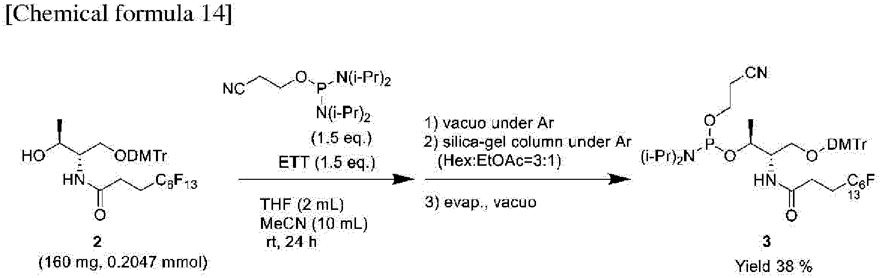

The compound 2 (160 mg, 0.20 mmol) dissolved in a mixed solvent of THF/acetonitrile (2 mL/2 mL) was added dropwise under an argon atmosphere to a solution containing 2-cyanoethyl N,N,N'N'-tetraisopropylphosphordiamidite (93 mg, 0.31 mmol) and ETT (40 mg, 0.31 mmol) dissolved in dry acetonitrile (8 mL). The resulting reaction mixture was stirred at room temperature for 24 hours, and the solvent was then evaporated under reduced pressure. The resulting crude product was purified by silica gel column chromatography under an argon atmosphere, using deaerated hexane /ethyl acetate (3:1 (volumetric ratio)) as the mobile phase. This enabled isolation of a compound 3 (DMTr-protected D-aTNA-N[FC8]) (yield: 38%) in the form of a colorless oil.

### Compound 3:

¹H NMR (400 MHz, CDCl₃) δ 7.41 to 7.20 (m, 9H), 6.82 to 6.79 (m, 4H), 5.94 (d, 9.1 Hz, 1H), 4.37 to 4.32 (m, 1H), 4.20 to 4.14 (m, 1H), 3.77 (s, 6H), 3.55 to 3.45 (m, 4H), 3.25 to 3.12 (m, 2H), 2.59 to 2.31 (m, 6H), 1.24 to 0.97 (m, 15H).

¹⁹F NMR (376 MHz, CDCl₃) δ -80.7 (s, 3F), -114.5 (s, 2F), -121.8 (s, 2F), -122.8 (s, 2F), -123.4 (s, 2F), -126.0 (s, 2F).

Using the synthesized D-aTNA-N[FC8], the phosphoramidite method was used in the same manner as Synthesis Example 1 to synthesize a nucleic acid having the structure shown below (D-aTNA-N1[FC8]: in the formula, the black dots indicate bonding sites).

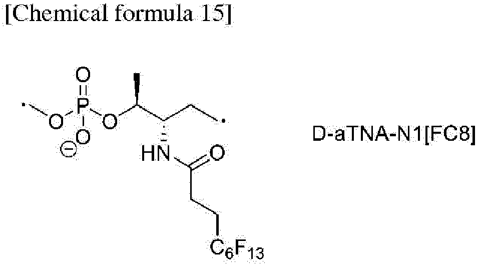

### [Example 1]

The cellular uptake efficiency of a fluorescein-modified nucleic acid containing the C₈-PFC group and a fluorescein-modified nucleic acid that did not contain the Cs-PFC group was investigated by quantitative analysis using flow cytometry.

### <Synthesis of Fluorescein-Modified Nucleic Acid>

A fluorescein-modified nucleic acid (ssDNA-FAM) having fluorescein introduced at the 3'-terminal of a single-stranded DNA (G-quadruplex DNA) comprising SEQ ID NO: 1 (5'-AGGGTTAGGGTTAGGGTTAGGG-3') was synthesized using a DNA synthesizer. Subsequently, two molecules of the N[C₈-PFC] amidite synthesized above were linked to the 5'-terminal of the obtained ssDNA-FAM by manual synthesis inside a glove box, thus obtaining an ssDNA-FAM having a cell membrane-permeable group containing two C₈-PFC groups linked in tandem to the 5'-terminal (namely, PFC-ssDNA-FAM).

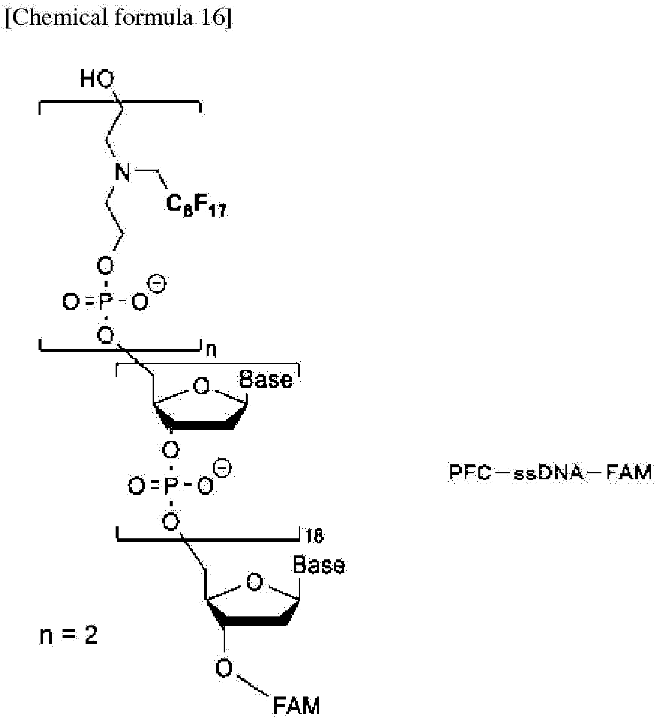

### <Preparation of Sample Solutions>

Solutions of ssDNA-FAM (control nucleic acid) and PFC-ssDNA-FAM (fluorinated nucleic acid) prepared in DMEM medium (low-glucose, 10% FBS, 1% mixed solution of penicillin-streptomycin) with concentrations of 1.0 µM were used as sample solutions. The 1.0 µM solution of ssDNA-FAM was used as a negative control, and a mixed solution containing the ssDNA-FAM and a commercially available transfection agent (Lipofectamine LTX, manufactured by Thermo Fisher Scientific Inc.) was used as a positive control. The mixing ratio and amounts used of the various reagents of the Lipofectamine LTX were in accordance with the standard protocol prescribed by the manufacturer. DMEM medium (low-glucose, 10% FBS, 1% mixed solution of penicillin-streptomycin) containing no introduced nucleic acid was used for blank measurements.

### <Evaluation of Cell Membrane Permeability by Flow Cytometry>

The medium of HeLa cells that had been precultured at 37°C for 24 hours was replaced with one of the sample solutions, and following culturing at 37°C for 1, 6 or 24 hours, the cell membrane permeability was evaluated. Following culturing for each of the prescribed time periods, the cell surfaces were washed three times with PBS (phosphate-buffered physiological saline solution), and the cells were then detached with trypsin-EDTA 0.05% (manufactured by Gibco Inc.) and collected. Subsequently, the collected cells were supplied to a flow cytometry system (Guava easyCyte (a registered trademark) 8), and the green fluorescence (488 nm) used for detecting the fluorescent dye fluorescein (FAM) introduced into the synthesized nucleic acid was measured.

Cells prepared without adding the fluorescein-modified nucleic acid to the medium were used as a blank, cells prepared by adding only the control nucleic acid (ssDNA-FAM) to the medium were used as a negative control, and cells prepared by adding both the control nucleic acid and Lipofectamine LTX to the medium were used as a positive control.

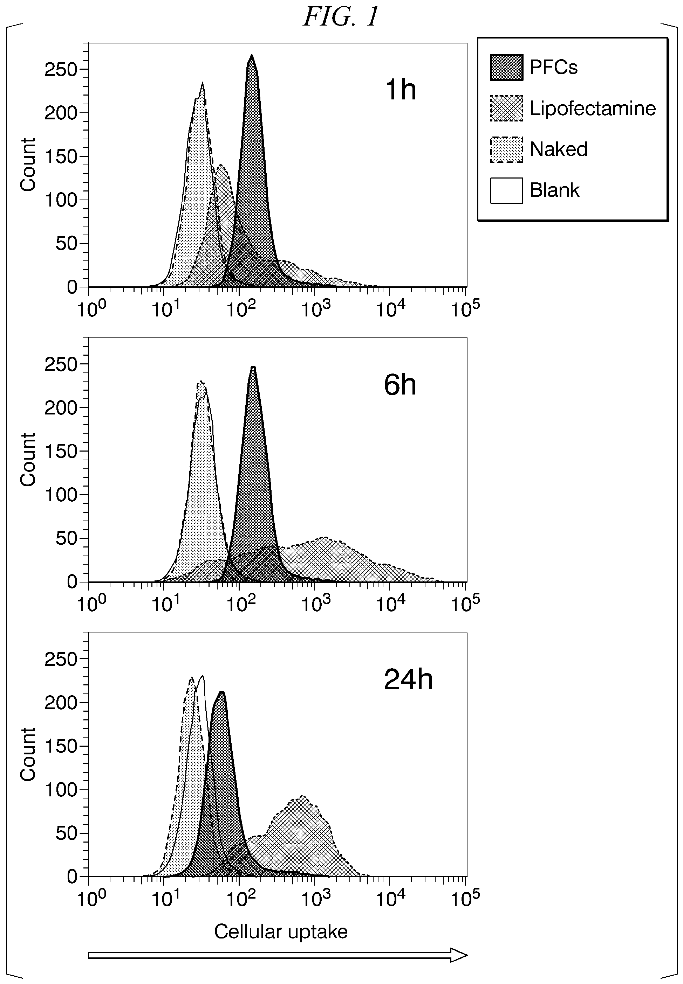

The fluorescent intensity distribution of each cell type after each culturing time period is illustrated in FIG. 1. In the figure, the label "Blank" indicates the results for the cells prepared without adding a fluorescein-modified nucleic acid to the medium, the label "Naked" indicates the results for the cells prepared by adding only the control nucleic acid (ssDNA-FAM) to the medium, the label "PFCs" indicates the results for the cells prepared by adding only the fluorinated nucleic acid (PFC-ssDNA-FAM comprising two molecules of N[C₈-PFC] amidite linked to the ssDNA-FAM) to the medium, and the label "Lipofectamine" indicates the results for the cells prepared by adding both the control nucleic acid and Lipofectamine LTX to the medium. As illustrated in FIG. 1, after culturing for any period of 1 to 24 hours, the cells prepared by adding only the control nucleic acid to the medium (Naked) only exhibited a fluorescent intensity of a similar level to the cells prepared without adding a fluorescein-modified nucleic acid to the medium (Blank), indicating almost no introduction of the nucleic acid into the cells. Further, in the case of the cells prepared by adding both the control nucleic acid and Lipofectamine LTX to the medium (Lipofectamine), although introduction of the nucleic acid into the cells was confirmed, the variation in the fluorescent intensity per cell was large. In contrast, in the case of the cells prepared by adding only the fluorinated nucleic acid to the medium, the fluorescent intensity distribution exhibited an extremely sharp peak, confirming that the nucleic acid had been efficiently introduced into almost all of the cells. These results suggested that the nucleic acid having an introduced cell membrane-permeable group containing a perfluoroalkyl group had superior cell membrane permeability to the widely used transfection reagent.

Lipofectamine is currently a widely used transfection reagent, but requires the culture solution to be serum-free, and also requires the added medium to be replaced every few hours. Further, not only is it necessary to prepare a mixture of solutions for each experiment, but the variation in the permeability results for each experiment also tends to be large. In contrast, the nucleic acid for transfection according to the present invention containing a perfluoroalkyl group does not require a serum-free culture medium and does not require medium replacement, meaning it offers excellent properties as a reagent for introducing the nucleic acid into a cell.

### [Example 2]

The cell survival rate of a fluorinated nucleic acid (PFC-ssDNA-FAM) synthesized in a similar manner to Example 1 using a single-stranded DNA (5'-TTTTTCAGTTGACCATATA-3', SEQ ID NO: 8) was measured by MTT assay, and the cytotoxicity was measured by LDH assay. Cells prepared without adding the fluorescein-modified nucleic acid to the medium were used as a blank, cells prepared by adding only the control nucleic acid (ssDNA-FAM) to the medium were used as a negative control, and cells prepared by adding both the control nucleic acid and Lipofectamine LTX to the medium were used as a positive control.

HeLa cells were inoculated into a 96-well culture plate at a concentration of 1.0 × 10⁴ cells/well, subjected to adhesion and proliferation and then cultured for 24 hours, and the fluorescein-modified nucleic acid (fluorinated nucleic acid or control nucleic acid) was then added to the medium in sufficient amount to achieve a final concentration of 1 µM, and culturing was conducted for 24, 48 or 72 hours. Following culturing, the cell survival rate was measured by MTT assay, and the cytotoxicity was measured by LDH assay. The MTT assay was measured using a commercially available MTT cell count measurement kit (manufactured by Nacalai Tesque, Inc.) in accordance with the provided protocol. The LDH assay was measured using a commercially available cytotoxicity measurement kit (manufactured by Dojindo Laboratories Co., Ltd.) in accordance with the provided protocol.

The relative cell survival rate (expressed as a % relative to a value of 100% for the cells of the blank) representing the measurement results of the MTT assays are shown in Table 1, and the cytotoxicity rate (%) (([measured value of measurement target cells] - [measured value for blank]) / [measured value of control with all cells dead] - [measured value for blank]) × 100%) representing the measurement results of the LDH assays are shown in Table 2. In the tables, the label "Blank" indicates the results for the cells prepared without adding a fluorescein-modified nucleic acid to the medium, the label "Naked" indicates the results for the cells prepared by adding only the control nucleic acid to the medium, the label "PFCs" indicates the results for the cells prepared by adding only the fluorinated nucleic acid to the medium, and the label "Lipofectamine" indicates the results for the cells prepared by adding both the control nucleic acid and Lipofectamine LTX to the medium.

**[Table 1]**

| | Cell survival rate (%) | | | |
|---|---|---|---|---|
| Culture time | Blank | Naked | PFCs | Lipofectamine |
| 24 hours | 100 | 102.6 | 103.7 | 62.4 |
| 48 hours | 100 | 97.7 | 93.6 | 36.6 |
| 72 hours | 100 | 97.6 | 94.8 | 44.6 |

**[Table 2]**

| | Cytotoxicity rate (%) | | | |
|---|---|---|---|---|
| Culture time | Blank | Naked | PFCs | Lipofectamine |
| 24 hours | 0 | -0.6 | -1.9 | 35.9 |
| 48 hours | 0 | -2.7 | -3.0 | 35.1 |
| 72 hours | 0 | -2.8 | -5.1 | 54.8 |

As is evident from Table 1, in the results of the MTT assays, there was little difference in the relative cell survival rate between the cells to which only the fluorinated nucleic acid was added and the cells to which only the control nucleic acid was added. On the other hand, as illustrated in Table 2, the results for the LDH assays revealed that when the control nucleic acid and the Lipofectamine LTX were both added to the medium, the cytotoxicity rate after 24 or 48 hours of culturing was about 35%, with this value rising to about 55% after 72 hours. In contrast, the cytotoxicity rate for the cells prepared by adding only the fluorinated nucleic acid to the medium was approximately 0%. These results confirmed that, unlike those cases where lipofectamine is used, the nucleic acid for transfection according to the present invention exhibits extremely low cytotoxicity.

### [Example 3]

The phosphoramidite containing a C₈-PFC group synthesized in Synthesis Example 1 (N[C₈-PFC] amidite) was linked to an siRNA targeting the Cyclin B1 gene to prepare an siRNA with a linked structure (A4), and the knock-down efficiency of this siRNA was investigated.

### <Preparation of siRNA>

The siRNA for targeting the Cyclin B1 gene used the sense strand and antisense strand comprising the base sequences shown in Table 3 (Non-Patent Document 7).

**[Table 3]**

| | Base sequence | SEQ ID NO: |
|---|---|---|
| Cyc-B1 sense | GGCGAAGAUCAACAUGGCATT | 2 |
| Cyc-B1 antisense | UGCCAUGUUGAUCUUCGCCTT | 3 |

A fluorine-modified sense strand was synthesized by coupling two molecules of the N[C₈-PFC] amidite to the 5'-terminal of the sense strand. The fluorine-modified sense strand and the unmodified antisense strand, each of which had been adjusted to a concentration of 8 µM using a buffer (0.1 M NaHPO₄, 0.1 M NaH₂PO₄, 1.0 M NaCl, pH 6.99), were denatured at 95°C for 10 minutes, and then cooled 5°C every 5 minutes, thus forming a double strand and yielding a fluorine-modified siRNA (C₈-PFC-modified siRNA).

### <Knock-Down Assay 1>

The medium of precultured HeLa cells was replaced with an siRNA solution prepared by diluting the C₈-PFC-modified siRNA with a culture medium to achieve a final concentration of 40 nM. As a control, the medium of precultured HeLa cells was replaced with the same amount of culture medium as the above siRNA solution. After 48 hours had elapsed from the medium replacement, the RNA was extracted from each set of cells in accordance with the protocol of an RNA extraction kit (RNAiso Plus, manufactured by Takara Bio Inc.).

Using the thus obtained total RNA as a template, real-time PCR was performed in accordance with the protocol of an RT-PCR kit ("One Step SYBER PrimeScript PLUS RT-PCR Kit", manufactured by Takara Bio Inc.), and the level of expression (amount of mRNA) of the Cyclin B1 gene was measured. As an internal standard, the level of expression of the GAPDH gene was also measured as a housekeeping gene. For the primers, a primer set for Cyclin B1 gene amplification (Primer Set ID CCNB1 (HA303687), manufactured by Takara Bio Inc.) or a primer set for GAPDH gene amplification (primer Set ID GAPDH (HA067812), manufactured by Takara Bio Inc.) was used at a final concentration of 0.2 µM.

Using the amount of total RNA extracted from the control cells as a reference, the knock-down efficiency was calculated from the amount of mRNA of the Cyclin B1 gene amplified from the total RNA extracted from the siRNA-introduced cells. More specifically, the amount of total RNA extracted from the control cells was subjected to serial dilution, real-time PCR was conducted, a calibration curve was created from the resulting amplification efficiency values, and this calibration curve was used to calculate the relative value of mRNA of the Cyclin B1 gene in the siRNA-introduced cells ([amount of mRNA of Cyclin B1 gene in siRNA-introduced cells] / [amount of mRNA of Cyclin B1 gene in control cells] × 100%). The mRNA amplification efficiency for the GAPDH gene was confirmed as having no difference between the total RNA extracted from the control cells and the total RNA extracted from the siRNA-introduced cells.

**[Table 4]**

| | Relative value for mRNA of Cyclin B1 gene [%] |
|---|---|
| Control cells | 100 |
| C8-PFC-modified siRNA-introduced cells | 65 |

As is evident from Table 4, in the cells having the introduced C₈-PFC-modified siRNA, the level of expression of the Cyclin B1 gene was suppressed by more than 30%, confirming that the C₈-PFC-modified siRNA had undergone transfection into the target cell, and had functioned normally as an siRNA inside the cells, enabling suppression of expression of the Cyclin B1 gene.

### <Knock-Down Assay 2>

The medium of precultured HeLa cells was replaced with an siRNA solution prepared by diluting the C₈-PFC-modified siRNA with a culture medium to achieve a final concentration of 100 nM. As a control, the medium of precultured HeLa cells was replaced with the same amount of culture medium as the above siRNA solution. After 24 hours had elapsed from the medium replacement, the RNA was extracted from each set of cells in accordance with the protocol of an RNA extraction kit (RNAiso Plus, manufactured by Takara Bio Inc.).

Using the same procedure as that described above in the section entitled <Knock-Down Assay 1>, real-time PCR was performed using the obtained total RNA as a template, and the level of expression (amount of mRNA) of the Cyclin B1 gene was measured.

Using the amount of total RNA extracted from the blank cells (cells to which the fluorescein-modified nucleic acid was not added) as a reference, the knock-down efficiency was calculated from the amount of mRNA of the Cyclin B1 gene amplified from the total RNA extracted from the siRNA-introduced cells. More specifically, the amount of total RNA extracted from the blank cells was subjected to serial dilution, real-time PCR was conducted, a calibration curve was created from the resulting amplification efficiency values, and this calibration curve was used to calculate the relative value of mRNA of the Cyclin B1 gene in the siRNA-introduced cells ([amount of mRNA of Cyclin B1 gene in siRNA-introduced cells] / [amount of mRNA of Cyclin B1 gene in blank cells] × 100%). The mRNA amplification efficiency for the GAPDH gene was confirmed as having no difference between the total RNA extracted from the blank cells and the total RNA extracted from the siRNA-introduced cells.

As a control, a C₈-PFC-modified siRNA comprising the base sequences shown in Table 5, including siRNA and a sense strand and antisense strand not targeting the Cyclin B1 gene (a scramble sequence), was introduced and compared.

**[Table 5]**

| | Base sequence | SEQ ID NO: |
|---|---|---|
| scramble sense | CGGAAGAGUAACUCACGGATT | 4 |
| scramble antisense | UCCGUGAGUUACUCUUCCGTT | 5 |

As illustrated in Table 6, in the case of the cells having the introduced Cs-PFC-modified siRNA, the level of expression of the Cyclin B1 gene was suppressed by about 30% (n=3). These results confirmed that the C₈-PFC-modified siRNA had undergone transfection into the target cell, and had functioned normally as an siRNA inside the cells, enabling suppression of expression of the Cyclin B1 gene.

**[Table 6]**

| | Relative value for mRNA of Cyclin B1 gene [%] | Standard deviation |
|---|---|---|
| Blank cells | 100 | |
| Unmodified Cyclin B1 targeting siRNA-introduced cells | 113 | 7.6 |
| C8-PFC-modified scramble sequence siRNA-introduced cells | 101 | 5.2 |
| C8-PFC-modified Cyclin B1 targeting siRNA-introduced cells | 71 | 4.5 |

### [Example 4]

With the exception of using the D-aTNA-N1[FC8] amidite synthesized in Synthesis Example 2 instead of the N[C₈-PFC] amidite synthesized in Synthesis Example 1, a nucleic acid synthesis reaction was conducted in the same manner as Example 1, enabling the synthesis of a fluorescein-modified nucleic acid (A1-ssDNA-FAM) having cell membrane-permeable groups containing the structure (A1) linked in tandem.

### <Evaluation of Cell Membrane Permeability by Flow Cytometry>

Using the same procedure as that described above in Example 1 in the section entitled <Evaluation of Cell Membrane Permeability by Flow Cytometry>, the medium of HeLa cells that had been precultured at 37°C for 24 hours was cultured in a medium containing the A1-ssDNA-FAM, and when the collected cells were supplied to flow cytometry, a fluorescent intensity distribution with an extremely sharp peak was obtained in a similar manner to that shown in FIG. 1 for the cells prepared by adding PFC-ssDNA-FAM to the medium. This sharp fluorescent intensity distribution indicates efficient introduction of the A1-ssDNA-FAM into the cells.

### <Knock-Down Assay>

With the exception of using the D-aTNA-N1[FC8] amidite synthesized in Synthesis Example 2 instead of the N[C₈-PFC] amidite, a nucleic acid synthesis reaction was conducted in the same manner as Example 2, yielding a fluorine-modified siRNA (A1-modified siRNA) modified with a cell membrane-permeable group containing the structure (A1). In the cells containing the introduced A1-modified siRNA, the level of expression of the Cyclin B1 gene was suppressed in a similar manner to that observed for the cells containing the introduced C₈-PFC-modified siRNA.

### [Synthesis Example 3]

A nucleic acid having the structure (A2) wherein R⁰ is a group represented by general formula (f-1) in which n1 is 2 and Rf^{P} represents a perfluorohexyl group was synthesized.

A phosphoramidite (L-aTNA-N[FC8]) having the structure (A2) wherein R⁰ is a group represented by general formula (f-1) in which n1 is 2 and Rf^{P} represents a perfluorohexyl group was synthesized as shown below.

### (1) Amidite Condensation

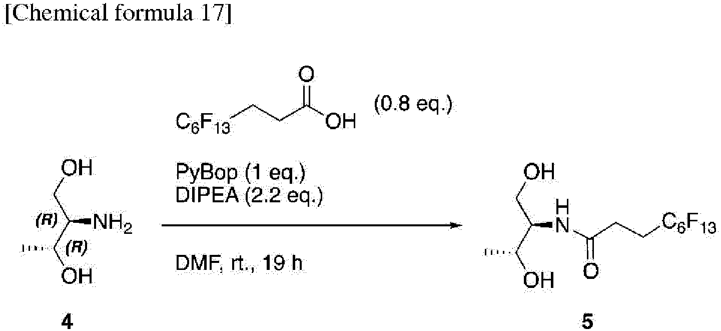

DIPEA (1.2 mL, 2.2 equivalents) and a compound 4 (0.32 g, 3.0 mmol) were added to 4,4,5,5,6,6,7,7,8,8,9,9,9-tridecafluorononanoic acid (0.96 g, 2.4 mmol, 0.8 equivalents) and PyBOP (1.6 g, 3.0 mmol) dissolved in 18 mL of DMF to complete preparation of the reaction solution under an argon atmosphere. The thus obtained reaction solution was stirred at room temperature for 19 hours. Subsequently, the reaction mixture was quenched with water (45 mL) and then extracted twice with hexane / ethyl acetate (3:7 (volumetric ratio)). The organic fractions were combined, washed with water, dried over Na₂SO₄ and filtered, and the solvent was then removed under vacuum. The resulting crude product was purified by silica gel column chromatography (hexane : ethyl acetate = 20:30 (volumetric ratio)), yielding a compound 5 (0.85 g, 2.4 mmol, yield: 60%).

### Compound 5:

¹H NMR (400 MHz, Acetone-D6) δ 4.06 (qd, J = 6.4, 2.7 Hz, 1H), 3.81 (tt, J = 8.8, 2.9 Hz, 1H), 3.65 to 3.57 (m, 2H), 2.69 to 2.50 (m, 4H), 1.11 (d, J = 6.4 Hz, 3H).

¹⁹F NMR (376 MHz, Acetone-D6) δ -81.6 (s, 3F), -114.8 (s, 2F), -122.3 (s, 2F), - 123.3 (s, 2F), -124.0 (s, 2F), -126.7 (s, 2F).

### (2) DMTr Protection

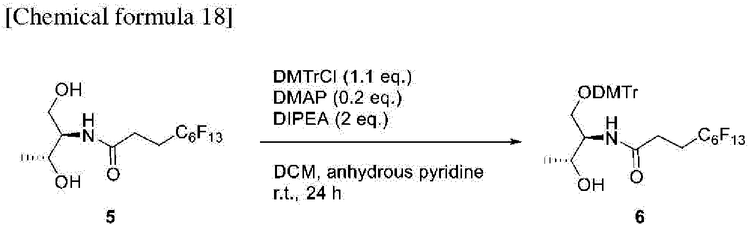

The compound 5 (0.38 g, 0.79 mmol 1.0 equivalents) was dissolved in 3.0 mL of anhydrous pyridine, and the solution was stirred over ice for 10 minutes. DIPEA (0.3 mL, 1.6 mmol, 2.0 equivalents) was then added, 3.0 mL of a DCM solution containing dissolved DMTrCl (0.31 mg, 0.87 mmol, 1.1 equivalents) and DMAP (0.15 mg, 0.11 mmol, 0.2 equivalents) was added, and the resulting mixture was stirred for 24 hours. Subsequently, the reaction mixture was diluted with 20 mL of DCM, and then washed twice with 20 mL samples of a saturated ammonium chloride aqueous solution, once with 20 mL of a saturated sodium bicarbonate aqueous solution, once with 20 mL of water, and then once with 20 mL of a saturated saline solution. The organic fraction was then dried over Na₂SO₄ and filtered, and the solvent was removed under reduced pressure. The resulting crude product was purified by silica gel column chromatography (hexane : ethyl acetate = 70:30 (volumetric ratio)), yielding a compound 6 (0.49 g, 0.62 mmol, yield: 79%).

### Compound 6:

¹H NMR (400 MHz, Acetone-D6) δ 7.37 to 7.14 (m, 9H), 6.87 to 6.80 (m, 4H), 4.08-3.99 (m, 2H), 3.75 (s, 6H), 3.21 (dd, J = 8.9, 6.0 Hz, 1H), 3.09 (dd, J = 8.9, 6.0 Hz, 1H), 2.65 to 2.47 (m, 4H), 1.06 (d, J = 6.4 Hz, 3H).

¹⁹F NMR (376 MHz, Acetone-D6) δ -81.6 (s, 3F), -114.9 (s, 2F), -122.4 (s, 2F), - 123.3 (s, 2F), -124.0 (s, 2F), -126.8 (s, 2F).

### (3) Amiditation

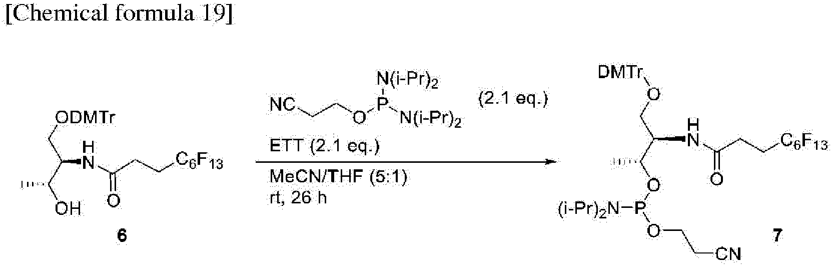

The compound 6 (254 mg, 0.32 mmol, 1.0 equivalents) dissolved in a mixed solvent of THF/acetonitrile (1 mL/1 mL) was added dropwise under an argon atmosphere to a solution containing 2-cyanoethyl N,N,N'N'-tetraisopropylphosphordiamidite (0.2 mL, 0.67 mmol 2.1 equivalents) and ETT (92 mg, 0.67 mmol, 2.1 equivalents) dissolved in dry acetonitrile (4 mL). The resulting reaction mixture was stirred at room temperature for 26 hours, and the solvent was then evaporated under reduced pressure. The resulting crude product was purified by silica gel column chromatography under an argon atmosphere, using deaerated hexane /ethyl acetate (2:1 (volumetric ratio)) as the mobile phase. This enabled isolation of a compound 7 (DMTr-protected L-aTNA-N[FC8]) (yield: 79%) in the form of a colorless oil.

### Compound 7:

¹H NMR (400 MHz, Acetone-D6) δ 7.36 to 7.28 (m, 9H), 6.91 to 6.87 (m, 4H), 4.38 to 4.27 (m, 2H), 3.79 (s, 6H), 3.62 to 3.53 (m, 4H), 3.27 to 3.16 (m, 2H), 2.62 to 2.52 (m, 6H), 1.23 to 1.12 (m, 15H).

¹⁹F NMR (376 MHz, Acetone-D6) δ -81.6 (s, 3F), -114.8 (s, 2F), -122.3 (s, 2F), - 123.3 (s, 2F), -124.0 (s, 2F), -126.7 (s, 2F).

Using the synthesized L-aTNA-N[FC8], the phosphoramidite method was used in the same manner as Synthesis Example 1 to synthesize a nucleic acid having the structure shown below (L-aTNA-N1[FC8]: in the formula, the black dots indicate bonding sites).

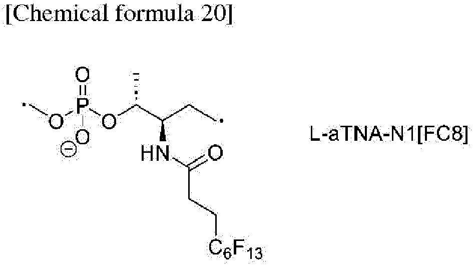

### [Example 5]

The L-aTNA-N1 [FC8] amidite synthesized in Synthesis Example 3 was linked to an siRNA targeting the luciferase gene to prepare an siRNA with a linked structure (A2).

### <Preparation of SiRNA>

A sense strand (Luc sense) and antisense strand (Luc antisense) comprising the base sequences shown in Table 7 were used for the siRNA for targeting the luciferase gene.

**[Table7]**

| | Base sequence | SEQ ID NO: |
|---|---|---|
| Luc sense | CUUACGCUGAGUACUUCGAAAUU | 6 |
| Luc antisense | UUUCGAAGUACUCAGCGUAAGUU | 7 |

A fluorine-modified sense strand (A2-modified sense strand) modified with a cell membrane-permeable group containing the structure (A2) was synthesized by coupling two molecules of the L-aTNA-N1 [FC8] amidite to the 5'-terminal of the sense strand. AFAM/A2-modified sense strand was also synthesized by introducing fluorescein at the 3'-terminal of this A2-modified sense strand.

RNA syntheses were conducted using commercially available reagents, various phosphoramidites (acetonitrile solutions, 0.1 M), and 5-ethylthio-1H-tetrazole (acetonitrile solution, 0.25 M) as an activator, using an NTS H-8 DNA/RNA synthesizer (manufactured by Nihon Techno Service Co., Ltd.).

The synthesized RNA was mixed with a diammonium citrate aqueous solution and a 3-hydroxypicolinic acid solution, and then identified by matrix-assisted laser desorption/ionization mass spectrometry. In Table 8, "L" means the L-aTNA-N1[FC8] amidite, and "FAM" means fluorescein. Further, "A2-Luc sense" means the A2-modified sense strand, and "FM/A2-Luc sense" means the FAM/A2-modified sense strand.

**[Table 8]**

| | Base sequence | Mw (M+H+) | |
|---|---|---|---|
| | | Calculated | Found |
| Luc antisense | 5'-UUUCGAAGUACUCAGCGUAAGUU-3' | 7309.351 | 7305.307 |
| Luc sense | 5'-CUUACGCUGAGUACUUCGAAAUU-3' | 7269.327 | 7265.045 |
| A2-Luc sense | 5'-LL-CUUACGCUGAGUACUUCGAAAUU-3' | 8351.725 | 8350.806 |
| FAM/A2-Luc sense | 5'-LL-CUUACGCUGAGUACUUCGAAAUU-FAM-3' | 8921.178 | 8918.371 |

The unmodified sense strand, the A2-modified sense strand or the FAM/A2-modified sense strand, and the unmodified antisense strand, each of which had been adjusted to a concentration of 8 µM, were denatured in a buffer (0.1 M NaHPO₄, 0.1 M NaH₂PO₄, 1.0 M NaCl, pH 6.99) at 95°C for 10 minutes, and then cooled 5°C every 5 minutes, thus forming a double strand and yielding an siRNA targeting the luciferase gene.

### <Cell Cultures>

Culturing of HeLa cells was conducted in the same manner as described above with the exception of adjusting the concentration of the mixed solution of penicillin-streptomycin in the culture medium to 1.0%.

### <Knock-Down Assay>

The medium of precultured HeLa cells was replaced with an siRNA solution prepared by diluting the D-aTNA[FC8]-modified siRNA with a culture medium to achieve a final concentration of 40 nM. As a control, the medium of precultured HeLa cells was replaced with the same amount of culture medium as the above siRNA solution. After 24 hours had elapsed from the medium replacement, a prescribed amount of Luciferase assay reagent was added in accordance with the protocol, and the amount of chemiluminescence was measured using a plate reader. The amount of chemiluminescence is an indicator of the level of luciferase expression.

Using the level of expression of luciferase (the amount of chemiluminescence) for the cells to which only PBS had been added as 100%, the results for the relative level of expression of luciferase for each of the cells is shown in Table 9. The relative level of expression of luciferase for the cells to which the D-aTNA[FC8]-modified siRNA had been added was 97%. The cells to which only siRNA had been added, which were used as the control, exhibited a result of 90%.

In contrast, the relative level of expression of luciferase for the cells to which a commercially available lipofectamine had been added together with the D-aTNA[FC8]-modified siRNA was 26%.

**[Table 9]**

| | Level of luciferase expression |
|---|---|
| PBS treatment only | 100% |
| siRNA | 90% |
| D-aTNA[FC8]-modified siRNA | 97% |
| D-aTNA[FC8]-modified siRNA + lipofectamine | 26% |

### [Synthesis Example 4]

Among the various nucleic acids having the structure (A1), a nucleic acid in which R⁰ represents an alkyl group of 10 carbon atoms substituted with one or more fluorine atoms, a nucleic acid in which R⁰ represents an alkyl group of 6 carbon atoms substituted with one or more fluorine atoms, a nucleic acid in which R⁰ represents an alkyl group of 8 carbon atoms not substituted with any fluorine atoms, and a nucleic acid in which R⁰ represents an alkyl group of 12 carbon atoms not substituted with any fluorine atoms were synthesized.

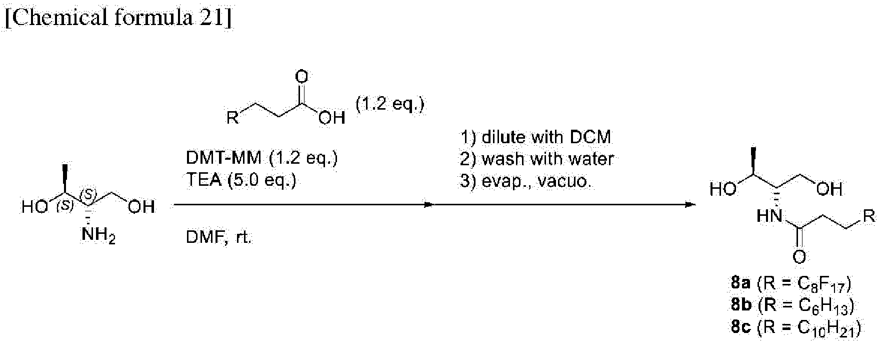

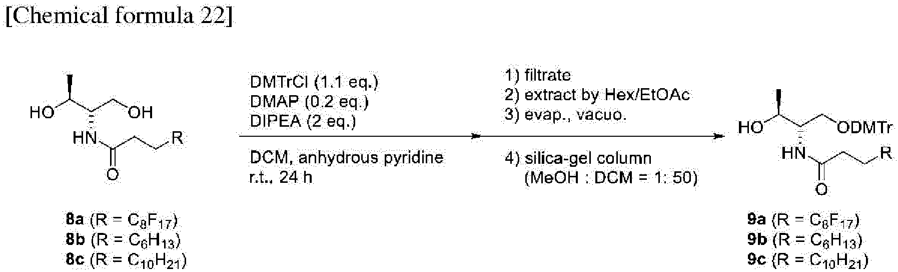

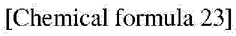

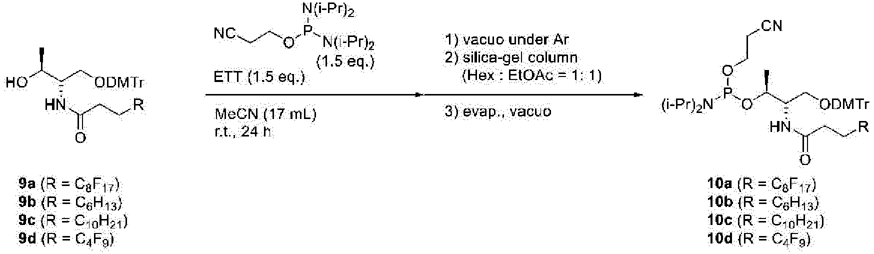

### <Synthesis of Phosphoramidite D-aTNA-N[FC10]>

A phosphoramidite (D-aTNA-N[FC10]) having the structure (A1) wherein R⁰ is a group represented by general formula (f-1) in which n1 is 2 and Rf^{P} represents a perfluorooctyl group was synthesized in the manner described below.

### (1) Amidite Condensation

D-threoninol (2.01 g, 19.02 mmol) and 4,4,5,5,6,6,7,7,8,8,9,9,10,10,11,11,11-tridecafluorononanoic acid (11.5 g, 22.8 mmol, 1.2 equivalents) was dissolved in 63 mL of DMF under a nitrogen atmosphere, triethylamine (17 mL, 5.0 equivalents) and DMT-MM (6.4 g, 1.2 equivalents) were added, and the thus obtained reaction solution was stirred at room temperature for 23 hours. Subsequently, the reaction mixture was diluted with DCM (100 mL), washed twice with water, dried over Na₂SO₄ and then filtered, and the solvent was then removed under vacuum, yielding a compound 8a (9.2 g, 15.8 mmol, yield: 83%).

### Compound 8a:

¹H-NMR (400 MHz, Acetone-D6) δ 7.07 (d, J = 7.7 Hz, 1H), 4.08 (dd, J = 6.4, 2.7 Hz, 1H), 3.81 (tt, J = 8.7, 2.9 Hz, 1H), 3.62 (t, J = 4.9 Hz, 2H), 2.68 to 2.53 (m, 4H), 1.12 (t, J = 6.2 Hz, 3H).

¹⁹F NMR (376 MHz, CDCl₃) δ -82.8 to -80.0 (3F), -116.8 to -111.8 (2F), -125.3 to -121.3 (10F), -128.7 to -125.0 (2F).

### (2) DMTr Protection

The diol 8a (5.75 g, 9.92 mmol) was dissolved in 25 mL of dry pyridine inside a 300 mL two-neck round-bottom flask, and the solution was cooled to 0°C. Next, diisopropylethylamine (3.6 mL, 20.7 mmol) was added to the mixture, and a solution of 4,4'-dimethoxytrityl chloride (3.7 g, 11.0 mmol) and 4-dimethylaminopyridine (0.19 g, 1.54 mmol) dissolved in 16 mL of dry dichloromethane was then added dropwise to the flask over a period of 20 minutes. The temperature of the mixture was raised to room temperature, the mixture was stirred for 17 hours, and the solvent was then removed under vacuum. The thus obtained crude product was purified by silica gel column chromatography using a mixed solvent of methanol/dichloromethane (1/50 (volumetric ratio)), and then dried under vacuum, yielding a compound 9a (8.29 g, 9.40 mmol, yield: 74%).

### Compound 9a:

¹H-NMR (400 MHz, Chloroform-D) δ 7.38 to 7.21 (m, 9H), 6.85 to 6.77 (m, 4H), 6.09 (d, J = 8.8 Hz, 1H), 4.12 (d, J = 6.2 Hz, 1H), 3.97 to 3.87 (m, 1H), 3.86 to 3.72 (m, 6H), 3.44 to 3.32 (m, 2H), 2.91 (d, J = 22.9 Hz, 1H), 2.60 to 2.38 (m, 4H), 1.25 to 1.08 (m, 3H).

¹⁹F NMR (376 MHz, CDCl₃) δ -80.6 (t, J = 9.8 Hz, 3F), -114.5 (s, 2F), -121.5 to -123.4 (m, 10F), -126.0 (s, 2F).

### (3) Amiditation

A solution of the compound 9a (3.52 g, 3.99 mmol) dissolved in acetonitrile (9 mL) was added dropwise under an argon atmosphere to a solution containing 2-cyanoethyl N,N,N'N'-tetraisopropylphosphordiamidite (1.9 mL, 5.99 mmol) and ETT (762 mg, 5.85 mmol) dissolved in dry acetonitrile (8 mL). The resulting reaction mixture was stirred at room temperature for 24 hours, and the solvent was then evaporated under reduced pressure. The resulting crude product was purified by silica gel column chromatography under an argon atmosphere, using deaerated hexane /ethyl acetate (3:1 (volumetric ratio)) as the mobile phase. This enabled isolation of a compound 10a (DMTr-protected D-aTNA-N[FC10]) (yield: 71%) in the form of a white solid.

### Compound 10a:

¹H-NMR (400 MHz, Chloroform-D) δ 7.41 to 7.15 (m, 9H), 6.82 to 6.76 (m, 4H), 4.38 to 4.08 (m, 2H), 3.83 to 3.67 (m, 6H), 3.56 to 3.40 (m, 3H), 3.25 to 3.03 (m, 2H), 2.62 to 2.29 (m, 6H), 1.29 to 1.08 (m, 12H), 1.02 to 0.97 (m, 3H).

¹⁹F-NMR (376 MHz, Chloroform-D) δ -80.6 (t, J = 9.8 Hz, 3F), -114.5 (d, J = 13.0 Hz, 2F), -121.6 to -123.4 (m, 10F), -126.0 (s, 2F).

³¹P-NMR (162 MHz, Chloroform-D) δ 148.9, 148.4.

### <Synthesis of Phosphoramidite D-aTNA-N[C8]>

A phosphoramidite (D-aTNA-N[C10]) having the structure (A1) wherein R⁰ is an octyl group was synthesized as shown below.

### (1) Amidite Condensation

Using octanoic acid instead of 4,4,5,5,6,6,7,7,8,8,9,9,9-tridecafluorononanoic acid as the carboxylic acid to undergo condensation, amidation was conducted in the same manner as described above. The reaction mixture was then diluted with dichloromethane, washed once with each of a saturated ammonium chloride aqueous solution, a saturated sodium bicarbonate aqueous solution and brine, dried over Na₂SO₄ and then filtered, and the solvent was then removed under vacuum. The resulting crude product was purified by silica gel column chromatography using ethyl acetate as the mobile phase, yielding a compound 8b (yield: 60%).

### Compound 8b:

¹H-NMR (400 MHz, Chloroform-D) δ 6.30 to 6.22 (m, 1H), 4.24 to 4.04 (m, 1H), 3.88 to 3.76 (m, 3H), 3.04 to 2.95 (m, 1H), 2.31 to 2.18 (m, 2H), 1.73 to 1.57 (m, 3H), 1.33 to 1.14 (m, 9H), 0.88 to 0.70 (m, 3H).

### (2) DMTr Protection

The diol 8b having a C₆H₁₃ side chain was subjected to DMTr modification using the same method as that described above for the diol 8a having a C₈F₁₇ side chain, thus synthesizing a compound 9b in the form of a colorless oil.

### Compound 9b:

¹H-NMR (400 MHz, Chloroform-D) δ 7.40 to 7.12 (m, 9H), 6.82 to 6.76 (m, 4H), 4.06 to 3.88 (m, 2H), 3.81 to 3.74 (m, 6H), 3.43 to 3.24 (m, 2H), 3.08 (d, J = 1.6 Hz, 1H), 2.21 (t, J = 7.7 Hz, 2H), 2.01 (d, J = 13.5 Hz, 2H), 1.30 to 1.23 (m, 12H), 1.13 (dd, J = 20.8, 6.2 Hz, 3H), 0.87 to 0.79 (m, 3H).

### (3) Amiditation

The DMTr-protected compound 9b having a C₆H₁₃ side chain was subjected to DMTr modification using the same method as that described above for the DMTr-protected compound 9a having a C₈F₁₇ side chain, and an amidite compound 10b (DMTr-protected D-aTNA-N[C8]) (yield: 55%) was isolated as a white solid.

### Compound 10b:

¹H-NMR (400 MHz, Chloroform-D) δ 7.47 to 7.07 (m, 9H), 6.83 to 6.71 (m, 4H), 4.38 to 4.16 (m, 1H), 3.83 to 3.74 (m, 6H), 3.59 to 3.41 (m, 3H), 3.26 to 3.04 (m, 2H), 2.49 to 2.29 (m, 1H), 2.23 to 2.10 (m, 2H), 1.26 to 1.07 (m, 24H), 1.02 to 0.97 (m, 1H), 0.87 to 0.78 (m, 3H).

### <Synthesis of Phosphoramidite D-aTNA-N[C12]>

A phosphoramidite (D-aTNA-N[C12]) having the structure (A1) wherein R⁰ is a dodecyl group was synthesized as shown below.

### (1) Amidite Condensation

Using dodecanoic acid instead of 4,4,5,5,6,6,7,7,8,8,9,9,9-tridecafluorononanoic acid as the carboxylic acid to undergo condensation, amidation was conducted in the same manner as described above. The reaction mixture was then diluted with dichloromethane, washed once with each of a saturated ammonium chloride aqueous solution, a saturated sodium bicarbonate aqueous solution and brine, dried over Na₂SO₄ and then filtered, and the solvent was then removed under vacuum. The resulting crude product was purified by silica gel column chromatography using hexane / ethyl acetate (1/1 (volumetric ratio)) as the mobile phase, yielding a compound 8c (yield: 76%).

### Compound 8c:

¹H-NMR (400 MHz, Chloroform-D) δ 6.16 (d, J = 5.0 Hz, 1H), 4.19 (q, J = 6.4 Hz, 1H), 3.86 (dd, J = 10.3, 2.1 Hz, 3H), 2.54 (d, J = 39.3 Hz, 2H), 2.26 (t, J = 7.5 Hz, 2H), 1.67 (q, J = 7.5 Hz, 2H), 1.37 to 1.26 (m, 20H), 1.21 (d, J = 6.4 Hz, 3H), 0.88 (t, J = 6.9 Hz, 3H).

### (2) DMTr Protection

The diol 8c having a C₁₀H₂₁ side chain was subjected to DMTr modification using the same method as that described above for the diol 8a having a C₈F₁₇ side chain, and a compound 9c was isolated in the form of a colorless oil (yield: 73%)

### Compound 9c:

¹H-NMR (400 MHz, Chloroform-D) δ 7.31 to 7.20 (m, 9H), 6.83 (dd, J = 8.9, 1.1 Hz, 4H), 6.07 (d, J = 8.7 Hz, 1H), 4.10 to 4.05 (m, 1H), 3.97 to 3.90 (m, 1H), 3.83 to 3.75 (m, 6H), 3.43 (q, J = 4.6 Hz, 1H), 3.27 (dd, J = 9.6, 3.7 Hz, 1H), 3.08 (d, J = 2.3 Hz, 1H), 2.24 to 2.17 (m, 2H), 1.69 to 1.61 (m, 2H), 1.31 to 1.20 (m, 18H), 1.12 (d, J = 6.4 Hz, 3H), 0.88 (t, J = 6.9 Hz, 3H).

### (3) Amiditation

The DMTr-protected compound 9c having a C₁₀H₂₁ side chain was subjected to amiditation using the same method as that described above for the DMTr-protected compound 9a having a C₈F₁₇ side chain, and a compound 10c (DMTr-protected D-aTNA-N[C12]) (yield: 61%) was isolated as a white solid.

### Compound 10c:

¹H-NMR (400 MHz, Chloroform-D) δ 7.41 (dd, J = 8.6, 1.3 Hz, 2H), 7.31 to 7.19 (m, 7H), 6.83 to 6.79 (m, 4H), 4.39 to 4.31 (m, 1H), 4.26 to 4.17 (m, 1H), 3.80 (t, J = 4.2 Hz, 6H), 3.56 to 3.42 (m, 4H), 3.24 to 3.05 (m, 2H), 2.44 to 2.30 (m, 2H), 2.19 to 2.15 (m, 2H), 1.29 to 1.19 (m, 25H), 1.16 to 1.10 (m, 12H), 0.87 (t, J = 7.0 Hz, 3H).

³¹P-NMR (162 MHz, Chloroform-D) δ 148.9, 148.7, 148.6.

### <Synthesis of Phosphoramidite D-aTNA-N[FC6]>

A phosphoramidite (D-aTNA-N[FC6]) having the structure (A1) wherein R⁰ is a group represented by general formula (f-1) in which n1 is 2 and Rf^{P} represents a perfluorobutyl group was synthesized in the manner described below.

### (3) Amiditation

A DMTr-protected compound 9d having a C₄H₉ side chain was subjected to amiditation using the same method as that described above for the DMTr-protected compound 9a having a C₈F₁₇ side chain, thus synthesizing an amidite compound 10d (DMTr-protected D-aTNA-N[FC6]) in the form of a white solid.

### Compound 10d:

¹H-NMR (400 MHz, Chloroform-D) δ 7.49 to 7.17 (m, 9H), 6.89 to 6.75 (m, 4H), 6.03 to 5.97 (m, 1H), 4.42 to 4.07 (m, 2H), 3.97 to 3.61 (m, 6H), 3.73 to 3.61 (m, 1H), 3.59 to 3.36 (m, 3H), 3.30 to 3.11 (m, 2H), 2.72 to 2.25 (m, 6H), 1.30 to 1.09 (m, 12H), 0.97 (q, J = 7.1 Hz, 3H).

¹⁹F-NMR (376 MHz, Chloroform-D) δ -80.7 to -81.1 (m, 3F), -114.5 to -115.2 (m, 2F), -124.2 to -124.4 (m, 2F), -125.6 to -126.4 (m, 2F).

³¹P-NMR (162 MHz, Chloroform-D) δ 148.9, 148.8, 148.4.

### <DNA Synthesis>

Using the four synthesized phosphoramidites and the phosphoramidite synthesized in Synthesis Example 3 (L-aTNA-N[FC8]), nucleic acids having the structures shown below (wherein in the formulas, the black dots indicate bonding sites) were synthesized by the phosphoramidite method in the same manner as that described for Synthesis Example 1.

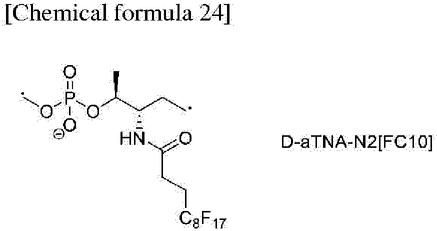

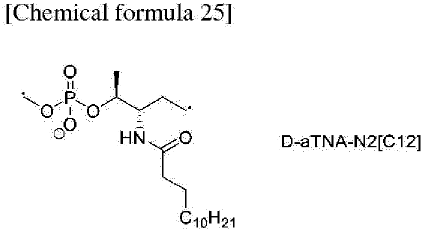

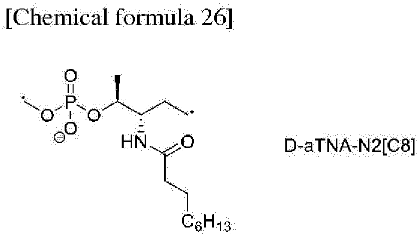

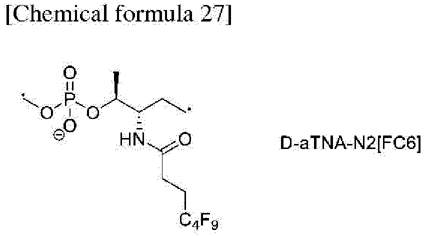

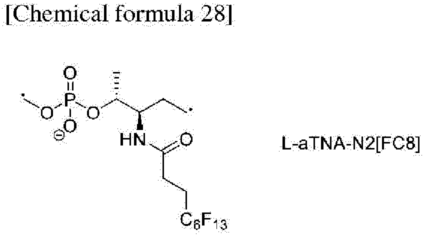

### [Example 6]

The cellular uptake efficiency of fluorescein-modified nucleic acids having any of the structures (A1) to (A4) and a fluorescein-modified nucleic acid having none of the structures (A1) to (A4) was investigated by quantitative analysis using flow cytometry. The fluorescein-modified nucleic acids having any of the structures (A1) to (A4) used the various phosphoramidites having a cell membrane-permeable group synthesized in the synthesis example described above (D-aTNA-N[C8], L-aTNA-N[FC8], D-aTNA-N[FC8], D-aTNA-N[FC10], and D-aTNA-N[C12]).

### <Synthesis of Fluorescein-Modified Nucleic Acid>

Nucleic acids having a fluorescein-modified nucleic acid (ssDNA-FAM) prepared by modifying a single-stranded DNA with fluorescein and a phosphoramidite having a cell membrane-permeable group linked to either the terminal of the nucleic acid portion of the fluorescein-modified nucleic acid (ssDNA-FAM) or within the nucleic acid portion (namely, AM-ssDNA-FAM nucleic acids) were synthesized.

Specifically, a fluorescein-modified nucleic acid (ssDNA-FAM) having fluorescein introduced at the 3'-terminal of a single-stranded DNA (SEQ ID NO: 8) was synthesized using a DNA synthesizer. Subsequently, either two molecules or five molecules of one of the phosphoramidites synthesized in the above synthesis example were linked to the 5'-terminal of the obtained ssDNA-FAM using a DNA synthesizer, thus obtaining an AM-ssDNA-FAM having two or five of the same phosphoramidite linked in tandem to the 5'-terminal.

Further, another fluorescein-modified nucleic acid (AM-ssDNA-FAM) was synthesized by using a DNA synthesizer to synthesize a single-stranded DNA having two molecules of a phosphoramidite with a fluoroalkyl group inserted between the thymine at position 9 and the thymine at position 10 of the single-stranded DNA (SEQ ID NO: 8) (namely, (5'-TTTTTCAGTR_{F}R_{F}TGACCATATA-3', wherein R_{F} represents a phosphoramidite-derived base having a fluoroalkyl group), and then introducing a fluorescein at the 3'-terminal of the thus obtained single-stranded DNA.

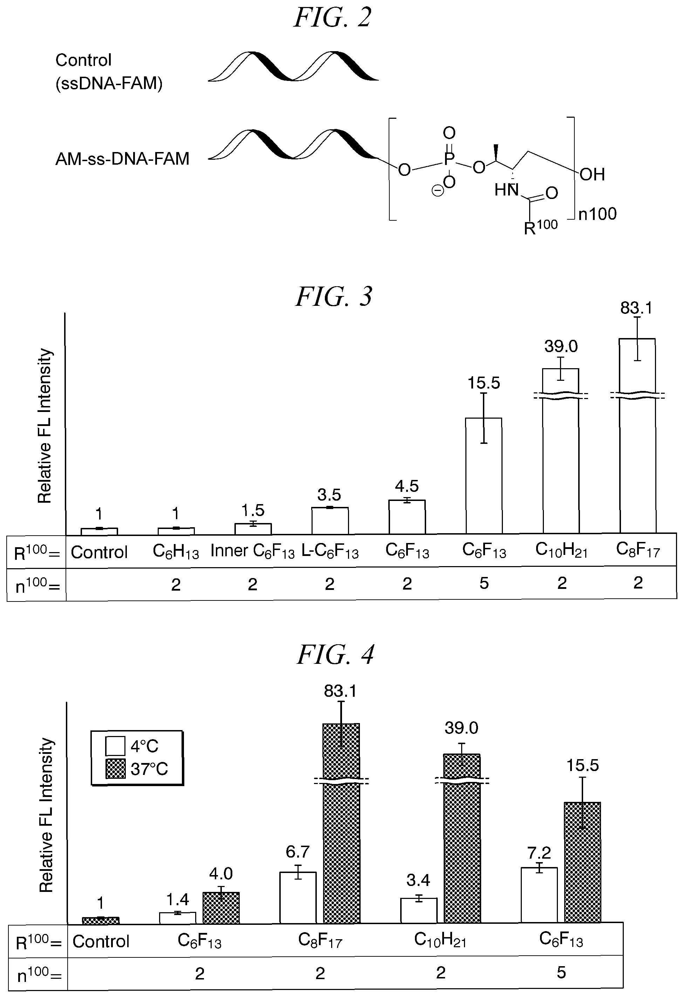

Schematic illustrations of the structures of the ssDNA-FAM (the control nucleic acid) and the AM-ssDNA-FAM having a cell membrane-permeable group at the 5'-terminal are shown in FIG. 2. In the structure of AM-ssDNA-FAM, "R¹⁰⁰" and "n100" are as listed in Table 10 shown below.

Specifically, the eight types of AM-ssDNA-FAM shown in Table 10 were synthesized, namely, D-aTNA-N2[FC6], D-aTNA-N2[C8], L-aTNA-N2[FC8], D-aTNA-N2[FC8], D-aTNA-N2[C12] and D-aTNA-N2[FC10], having two molecules of D-aTNA-N[FC6], D-aTNA-N[C8], L-aTNA-N[FC8], D-aTNA-N[FC8], D-aTNA-N[C12] and D-aTNA-N[FC10] respectively linked in tandem to the 5'-terminal of the ssDNA-FAM, and in addition, D-aTNA-N5[FC8] having five molecules of D-aTNA-N[FC8] linked in tandem to the 5'-terminal of the ssDNA-FAM, and D-aTNA-midN2[FC8] having two molecules of D-aTNA-N[FC8] inserted in tandem near the center of the ssDNA-FAM were also synthesized.

### <Evaluations of Physical Properties of Fluorescein-Modified Nucleic Acids>

For each of the fluorescein-modified nucleic acids, the hydrodynamic diameter in a PBS solution, the zeta potential in an aqueous solution, and the critical micelle concentration (CMC) (µM) in a PBS solution are listed in Table 10. In the table, the "Control" label represents the ssDNA-FAM (control nucleic acid).

The hydrodynamic diameter (nm) of each fluorescein-modified nucleic acid was measured by dissolving a sample of the fluorescein-modified nucleic acid in PBS to form a test sample solution of concentration 2 µM, and then conducting a measurement using a dynamic light-scattering (DLS) measurement device. In DLS, a laser light is irradiated onto a thin suspension, and the fluctuation in the scattered light across a fixed period is measured by detecting the back-scattered light from the microparticles in the suspension. Brownian motion is induced in the microparticles in the liquid by the thermally agitated solvent molecules, and the fluctuation in the scattered light is dependent upon the velocity of the Brownian motion of the particles, with smaller particles generating faster Brownian motion. In a DLS measurement, by detecting and correlating the change in the scattered light fluctuation across a fixed period (the correlation time), a decay curve known as an autocorrelation function is acquired. The diffusion coefficient in the Stokes-Einstein equation can be calculated from this autocorrelation function, and then converted to a particle diameter. Specifically, a test sample was prepared in a small-volume quartz batch cell (volume: 12 µL, ZEN2112, manufactured by Malvern Panalytical Ltd.), and the particle diameter was measured at 37°C using a DLS measurement system of wavelength 630 nm (Zetasizer Advance Series Pro (Red label), manufactured by Malvern Panalytical Ltd.). The same sample was measured three or more times, and the average particle diameter was calculated from the measurement results.

The zeta potential (mV) of each fluorescein-modified nucleic acid was measured by dissolving a sample of the fluorescein-modified nucleic acid in ultrapure water to form a test sample solution of concentration 10 µM, and then conducting a measurement using a DLS measurement device. The electrophoretic mobility of the particles dispersed in the liquid is dependent on the zeta potential of those particles. A laser light is irradiated onto the particles, the electrophoretic mobility is determined from the variation in the scattered light frequency, and the Henry equation is used to calculate the zeta potential. The zeta potential is an indicator of the electrostatic repulsive force of the particles, and is widely used as a method for evaluating dispersion stability and simply estimating the surface state of the particles. Specifically, a test sample was prepared in a disposable cell for measuring zeta potential (DTS1070, manufactured by Malvern Panalytical Ltd.), and the zeta potential was measured at 37°C using a DLS measurement system (Zetasizer Advance Series Pro (Red label), manufactured by Malvern Panalytical Ltd.).

The critical micelle concentration (CMC) of each fluorescein-modified nucleic acid was measured by dissolving samples of the fluorescein-modified nucleic acid in PBS to form test sample solutions of various concentrations, and then conducting measurements using a DLS measurement system (Zetasizer Advance Series Pro (Red label), manufactured by Malvern Panalytical Ltd.). The critical micelle concentration was calculated by counting the count rate for test samples across a wide range of concentrations using the DLS system. The test samples were measured at 37°C using a DLS measurement system of wavelength 630 nm, and the count rates across the broad range of concentrations were calculate from the measurement results. The count rate starts to increase when micelle formation begins. Accordingly, in the present invention and in this description, the concentration at which the count rate started to increase was deemed the CMC.

**[Table 10]**

| Fluorescein-modified nucleic acid | R¹⁰⁰ | n100 | Hydrodynamic diameter [nm] | Zeta potential [mV] | CMC [µM] |
|---|---|---|---|---|---|
| Control | | | <5 | -43±4 | N.A. |
| D-aTNA-N2[FC6] | C₄F₉ | 2 | <5 | | 1.27 |
| D-aTNA-N2[C8] | C₆H₁₃ | 2 | 66±5 | | 1.62 |
| D-aTNA-midN2[FC8] | Inner C₆F₁₃ | 2 | <5 | | 2.33 |
| L-aTNA-N2[FC8] | C₆F₁₃ | 2 | 50±7 | | 0.51 |
| D-aTNA-N2[FC8] | C₆F₁₃ | 2 | 64±15 | -67±5 | 0.64 |
| D-aTNA-N5[FC8] | C₆F₁₃ | 5 | 9±1 | | 0.33 |
| D-aTNA-N2[C12] | C₁₀H₂₁ | 2 | 66±14 | -82±4 | 0.38 |
| D-aTNA-N2[FC10] | C₈F₁₇ | 2 | 63±11 | | 0.21 |

### <Preparation of Sample Solutions>

Solutions prepared by dissolving the ssDNA-FAM (the control nucleic acid) and each of the various AM-ssDNA-FAM nucleic acids in DMEM culture medium at a concentration of 2.0 µM were used a sample solutions. The 2.0 µM solution of the ssDNA-FAM was used as a negative control. DMEM liquid medium was used for conducting a blank measurement.

### <Evaluation of Cell Membrane Permeability by Flow Cytometry>

The medium of HeLa cells that had been precultured at 37°C for 24 hours was replaced with one of the sample solutions, and following culturing at 37°C for four hours, the cell membrane permeability was evaluated. Following culturing for the prescribed time period, the cell surfaces were washed three times with PBS (phosphate-buffered physiological saline solution), and the cells were then detached with trypsin-EDTA 0.05% (manufactured by Gibco Inc.) and collected. Subsequently, the collected cells were supplied to a flow cytometry system (Guava easyCyte (a registered trademark) 8), and the green fluorescence (488 nm) used for detecting the fluorescent dye fluorescein (FAM) introduced into the synthesized nucleic acid was measured.

Cells prepared without adding a fluorescein-modified nucleic acid to the medium were used as a blank, and cells prepared by adding only the control nucleic acid (ssDNA-FAM) to the medium were used as a negative control.

The fluorescent intensity was measured for each cell type, and a relative fluorescent intensity was determined relative to a value of 1.0 for the fluorescent intensity of the cells prepared by adding only the control nucleic acid to the medium. The results are shown in FIG. 3. As illustrated in FIG. 3, the mean value for the relative fluorescent intensity for those cells having an introduced fluorescein-modified nucleic acid in which R¹⁰⁰ is either a perfluoroalkyl group or an alkyl group of 10 or more carbon atoms was 1.5 or higher, indicating a clearly stronger fluorescent intensity than the cells with the introduced control nucleic acid, and confirming that the cell membrane permeability of these fluorescein-modified nucleic acids was superior to that of the control nucleic acid. In contrast, the relative fluorescent intensity for the cells having an introduced fluorescein-modified nucleic acid in which R¹⁰⁰ is an alkyl group of 9 or fewer carbon atoms containing no fluorine atoms was 1.0, similar to that of the control nucleic acid.

### <Confirmation of Energy-Dependent Intracellular Delivery>

The medium of HeLa cells that had been precultured at 37°C for 24 hours was replaced with DMEM liquid medium, and culturing was conducted at 4°C for 0.5 hours. Subsequently, the medium was replaced with a sample solution, and after culturing at either 4°C or 37°C for 1.5 hours, the cell membrane permeability was evaluated. Evaluation of the cell membrane permeability was conducted in the same manner as that described above for the evaluation of cell membrane permeability by flow cytometry.

Cells prepared without adding any fluorescein-modified nucleic acid to the medium were used as a blank, and cells prepared by adding only the control nucleic acid (ssDNA-FAM) to the medium were used as a negative control.

The fluorescent intensity was measured for each cell type, and a relative fluorescent intensity was determined relative to a value of 1.0 for the fluorescent intensity of the cells prepared by adding only the control nucleic acid to the medium and then performing culturing at 37°C. The results are shown in FIG. 4. In the figure, the labels "Control", "R¹⁰⁰", and "n100" have the same meanings as Table 10. As illustrated in FIG. 4, the relative fluorescent intensity for those cells having an introduced fluorescein-modified nucleic acid in which R¹⁰⁰ is either a perfluoroalkyl group or an alkyl group of 10 or more carbon atoms was much lower when culturing was conducted at 4°C than when culturing was conducted at 37°C, indicating that uptake of the fluorescein-modified nucleic acid into the cells decreased upon culturing at 4°C. These results suggested that cellular uptake of the nucleic acids having the structures (A1) to (A4) was by energy-dependent intracellular delivery (endocytosis).

### [Synthesis Example 5]

Among the various nucleic acids having the structure (A4), a phosphoramidite having a structure in which R^{FE} in general formula (A4) is a perfluoroalkyl group of 6 carbon atoms (C₆F₁₃-: hereinafter referred to as a C₆-PFC group) and na represents 1 was synthesized, and using this phosphoramidite, a nucleic acid containing the C₆-PFC group was produced by the amidite method.

### <Synthesis of Phosphoramidite having C₆-PFC Group>

A phosphoramidite containing the C₆-PFC group represented by the formula shown below (namely, an N[C₆-PFC] amidite) was synthesized using the same method as Synthesis Example 1.

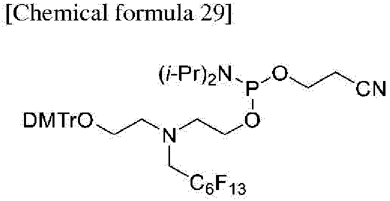

¹H-NMR (400 MHz, Chloroform- D) δ 7.45 to 7.39 (m, 2H), 7.35 to 7.26 (m, 6H), 7.23 to 7.17 (m, 1H), 6.85 to 6.79 (m, 4H), 3.83 to 3.52 (m, 6H), 3.79 (s, 6H), 3.79 (t, J = 16.6 Hz, 2H), 3.20 (t, J = 5.7 Hz, 2H), 2.94 (dt, J = 15.6, 5.9 Hz, 4H), 2.57 (t, J = 6.5 Hz, 2H), 1.16 (dd, J = 15.3, 6.8 Hz, 12H).

¹⁹F-NMR (376 MHz, Chloroform- D) δ -80.64 (t, J = 10.1 Hz, 3F), -117.31 to -117.72 (m, 2F), -121.86- to -121.91 (m, 2F), -122.55 to -122.94 (m, 2F), -123.32 to -123.40 (m, 2F), -125.93 to -126.06 (m, 2F).

³¹P-NMR (162 MHz, Chloroform- D) δ 148.35.

### [Synthesis Example 6]

Among the various nucleic acids having the structure (A4), a phosphoramidite having a structure in which R^{FE} in general formula (A4) is a perfluoro polyether group of 5 carbon atoms (CF₃-(OCF₂)₄-: hereinafter referred to as a C₅-PFPE group) and na represents 1 was synthesized, and using this phosphoramidite, a nucleic acid containing the C₅-PFPE group was produced by the amidite method.

### <Synthesis of Phosphoramidite having C₅-PFPE Group>

A phosphoramidite containing the C₅-PFPE group represented by the formula shown below (namely, an N[C₅-PFPE] amidite) was synthesized using the same method as Synthesis Example 1.

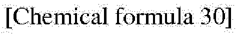

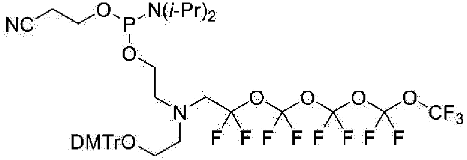

¹H-NMR (400 MHz, Chloroform- D) δ 7.44 to 7.39 (m, 2H), 7.35 to 7.27 (m, 6H), 7.23 to 7.17 (m, 1H), 6.89 to 6.75 (m, 4H), 3.82 to 3.45 (m, 6H), 3.79 (s, 6H), 3.27 (t, J = 11.1 Hz, 2H), 3.18 (t, J = 5.8 Hz, 2H), 2.93 (dt, J = 14.2, 6.0 Hz, 4H), 2.57 (tt, J = 6.5, 0.9 Hz, 2H), 1.15 (dd, J = 15.4, 6.8 Hz, 12H).

¹⁹F-NMR (376 MHz, Chloroform- D) δ -52.93 (p, J = 10.8 Hz, 2F), -54.85 (p, J = 10.1 Hz, 2F), -55.36 (qt, J = 9.4, 9.3 Hz, 2F), -56.76 (t, J = 8.8 Hz, 3F), -77.18 (td, J = 11.40, 11.39 Hz, 2F).

³¹P-NMR (162 MHz, Chloroform- D) δ 148.40.

### [Example 7]

The cellular uptake efficiency of the fluorescein-modified nucleic acid containing a C₈-PFC group was investigated by quantitative analysis using flow cytometry.

### <Synthesis of Fluorescein-Modified Nucleic Acid>

A fluorescein-modified nucleic acid (ssDNA-FAM) having fluorescein introduced at the 3'-terminal of a single-stranded DNA comprising SEQ ID NO: 8 was synthesized in the same manner as Example 1. Subsequently, 1, 2, 3, 4 or 5 molecules of the N[C₈-PFC] amidite synthesized in Synthesis Example 1 were linked to the 5'-terminal of the obtained ssDNA-FAM in the same manner as Example 1, thus obtaining an ssDNA-FAM having a cell membrane-permeable group containing 1, 2, 3, 4 or 5 Cs-PFC groups linked in tandem to the 5'-terminal (namely, PFC-ssDNA-FAM).

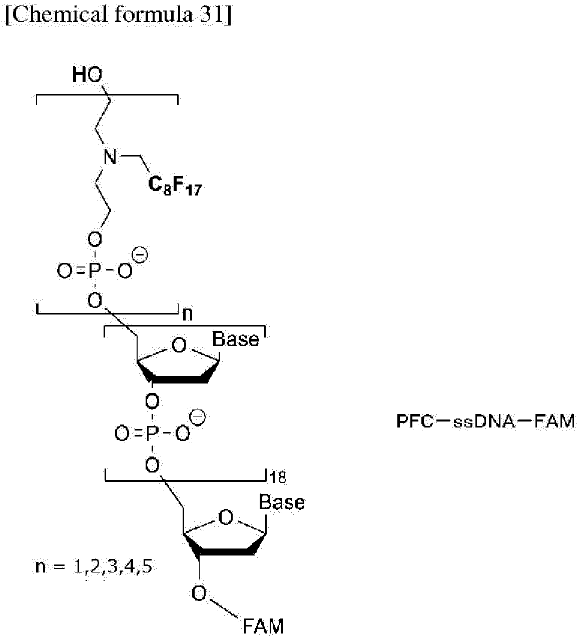

### <Preparation of Sample Solutions>

Solutions of ssDNA-FAM (the control nucleic acid) and PFC-ssDNA-FAM (the fluorinated nucleic acids) prepared in DMEM medium (low-glucose, 10% FBS, 1% mixed solution of penicillin-streptomycin) with concentrations of 1.0 µM were used as sample solutions. The DMEM medium (low-glucose, 10% FBS, 1% mixed solution of penicillin-streptomycin) was also used for blank measurements.

### <Evaluation of Cell Membrane Permeability by Flow Cytometry>

The cell membrane permeability of HeLa cells following culturing in each of the sample solutions was evaluated in the same manner as Example 1. Cells prepared without adding any fluorescein-modified nucleic acid to the medium were used as a blank.

The mean fluorescent intensity (MFI) for each cell solution after each culturing time period is illustrated in Table 11. In the table, the label "Blank" indicates the results for the cells prepared without adding a fluorescein-modified nucleic acid to the medium, whereas the labels "N[PFC]n-DNA" indicate the results for the cells prepared by adding ssDNA-FAM containing n added molecules of fluorinated nucleic acid to the medium. As illustrated in Table 11, for the results observed after one hour, the mean value for the fluorescent intensity of the fluorinated nucleic acids (PFC-ssDNA-FAM) reached a maximum when n=2. These results suggested that in the case of the C₈-PFC group, the most superior cell membrane permeability is achieved when two-molecule modification is performed.

**[Table 11]**

| Mean fluorescent intensity (MFI) | 1h | | | 6h | | | 24h | | |
|---|---|---|---|---|---|---|---|---|---|
| | Data1 | Data2 | Data3 | Data1 | Data2 | Data3 | Data1 | Data2 | Data3 |
| Blank | 32.8 | 32.4 | 32.4 | 32.5 | 31.6 | 36.4 | 31.8 | 31.9 | 32 |
| N[PFC]₀-DNA | 35.8 | 36.3 | 36.4 | 37.2 | 39 | 38.1 | 49 | 42.6 | 44.6 |
| N[PFC]₁-DNA | 46.7 | 45.5 | 43.1 | 60 | 58.3 | 57.3 | 60.6 | 59.9 | 60 |
| N[PFC]₂-DNA | 364 | 372 | 409 | 462 | 481 | 513 | 191 | 196 | 189 |
| N[PFC]₃-DNA | 136 | 139 | 140 | 360 | 318 | 369 | 242 | 244 | 223 |
| N[PFC]₄-DNA | 130 | 131 | 138 | 474 | 462 | 458 | 329 | 342 | 423 |
| N[PFC]₅-DNA | 138 | 124 | 130 | 489 | 521 | 520 | 418 | 470 | 473 |

### [Example 8]

The phosphoramidite containing a C₆-PFC group synthesized in Synthesis Example 5 (N[C₆-PFC] amidite) was linked to an siRNA targeting the Cyclin B1 gene to prepare an siRNA with a linked structure (A4), and the knock-down efficiency of this siRNA was investigated.

### <Preparation of siRNA>

The siRNA for targeting the Cyclin B1 gene used the sense strand and antisense strand comprising the base sequences shown in Table 3.

In a similar manner to Example 3, 1, 2, 3, 4 or 5 molecules of the N[C₆-PFC] amidite were coupled to the 5'-terminal of the sense strand to synthesize a fluorine-modified sense strand, and a double strand was then formed together with either an unmodified antisense strand or an antisense strand having one coupled molecule of the N[C₆-PFC] amidite, thus obtaining a fluorine-modified siRNA (C₆-PFC-modified siRNA).

### <Knock-Down Assay>

The medium of precultured HeLa cells was replaced with an siRNA solution prepared by diluting the C₆-PFC-modified siRNA with a culture medium to achieve a final concentration of either 10 nM or 100 nM. As a control, the medium of precultured HeLa cells was replaced with the same amount of culture medium as the above siRNA solution. After 48 hours had elapsed from the medium replacement, the RNA was extracted from each set of cells in the same manner as Example 3, RT-PCR was performed using the obtained total RNA as a template, and the level of expression (amount of mRNA) of the Cyclin B1 gene was measured.

In a similar manner to Example 3, the amount of total RNA extracted from the control cells was used as a reference, and the knock-down efficiency was calculated from the amount of mRNA of the Cyclin B1 gene amplified from the total RNA extracted from the siRNA-introduced cells. The relative value (%) for the level of expression of the Cyclin B1 gene in each of the cells, relative to a value of 100 for the level of expression of the Cyclin B1 gene in the blank (cells prepared by adding only the DMEM medium (low-glucose, 10% FBS, 1% mixed solution of penicillin-streptomycin), is shown in Table 12. In the table, "n" represents the number of N[C₆-PFC] amidites coupled to each strand.

**[Table 12]**

| sense (n) | 10 nM antisense n=0 | 100 nM antisense n=0 | 10 nM antisense n=1 | 100 nM antisense n=1 |
|---|---|---|---|---|
| 0 | 142.5 | 122.5 | - | - |
| 1 | 110.0 | 75.5 | 81.5 | 34.3 |
| 2 | 141.0 | 15.1 | 109.0 | 79.5 |
| 3 | 140.0 | 40.5 | 108.5 | 43.3 |
| 4 | 133.5 | 92.5 | 159.5 | 84.5 |
| 5 | 84.5 | 77.0 | 88.5 | 37.0 |

As illustrated in Table 12, when the final concentration of siRNA was 100 nM, the cells into which the C₆-PFC-modified siRNA had been introduced had a lower relative level of expression of the Cyclin B1 gene than the cells into which the unmodified siRNA had been introduced. In particular, in the cells containing 100 nM of the two molecule C₆-PFC-modified siRNA, the level of expression of the Cyclin B1 gene was suppressed by about 85% compared with the blank. These results confirmed that the C₆-PFC-modified siRNA had undergone transfection into the target cells, and had functioned normally as an siRNA inside the cells, enabling suppression of expression of the Cyclin B1 gene.

### [Example 9]

The cellular uptake efficiency of the fluorescein-modified nucleic acid containing a C₅-PFPE group was investigated by quantitative analysis using flow cytometry.

### <Synthesis of Fluorescein-Modified Nucleic Acid>

A fluorescein-modified nucleic acid (ssDNA-FAM) having fluorescein introduced at the 3'-terminal of a single-stranded DNA comprising SEQ ID NO: 1 was synthesized in the same manner as Example 1. Subsequently, two molecules of the N[C₅-PFPE] amidite synthesized in Synthesis Example 6 were linked to the 5'-terminal of the obtained ssDNA-FAM in the same manner as Example 1, thus obtaining an ssDNA-FAM having a cell membrane-permeable group containing two C₅-PFPE groups linked in tandem to the 5'-terminal (namely, PFPE-ssDNA-FAM).

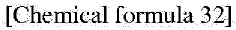

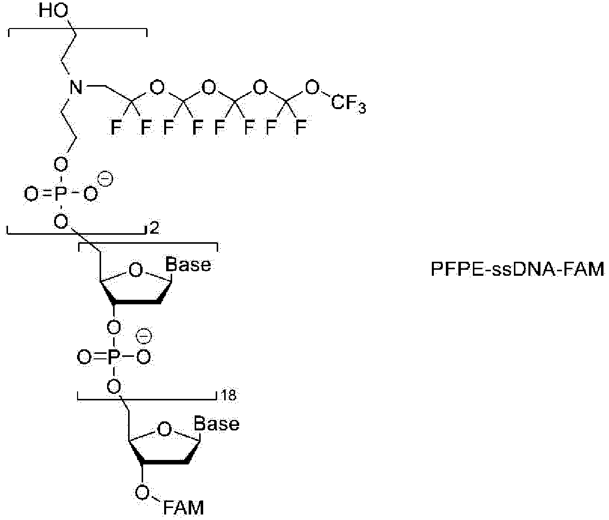

### <Preparation of Sample Solutions>

A solution of the PFPE-ssDNA-FAM (the fluorinated nucleic acid) prepared in DMEM medium (low-glucose, 10% FBS, 1% mixed solution of penicillin-streptomycin) with a concentration of 1.0 µM was used as a sample solution. DMEM medium (low-glucose, 10% FBS, 1% mixed solution of penicillin-streptomycin) was also used for blank measurements.

### <Evaluation of Cell Membrane Permeability by Flow Cytometry>

In a similar manner to Example 1, HeLa cells were cultured in each of the sample solutions at 37°C for 28 hours, and following culturing, the cell membrane permeability was evaluated. Cells prepared without adding the fluorescein-modified nucleic acid to the medium were used as a blank.

The mean fluorescent intensity for each cell solution after the culturing period is illustrated in Table 13. In the table, the label "Blank" indicates the results for the cells prepared without adding the fluorescein-modified nucleic acid to the medium, and the label "C₃-PFPE" indicates the results for the cells prepared by adding ssDNA-FAM containing two added molecules of the fluorinated nucleic acid to the medium. As illustrated in Table 13, the mean value for the fluorescent intensity of the fluorinated nucleic acid (PFPE-ssDNA-FAM) was approximately twice that of the blank, indicating that superior cell membrane permeability could be achieved by modifying the nucleic acid with the C₅-PFPE group.

**[Table 13]**

| | Blank | C₅-PFPE |
|---|---|---|
| Mean fluorescent intensity (MFI) | 44 | 94 |

### [Example 10]

The phosphoramidite containing a C₅-PFPE group synthesized in Synthesis Example 6 (N[C₅-PFPE] amidite) was linked to an siRNA targeting the Cyclin B1 gene to prepare an siRNA with a linked structure (A4), and the knock-down efficiency of this siRNA was investigated.

### <Preparation of siRNA>

The siRNA for targeting the Cyclin B1 gene used the sense strand and antisense strand comprising the base sequences shown in Table 3.

In a similar manner to Example 3, two molecules of the N[C₅-PFPE] amidite were coupled to the 5'-terminal of the sense strand to synthesize a fluorine-modified sense strand, and a double strand was then formed together with an unmodified antisense strand, thus obtaining a fluorine-modified siRNA (C₅-PFPE-modified siRNA).

### <Knock-Down Assay>

The medium of precultured HeLa cells was replaced with an siRNA solution prepared by diluting the C₅-PFPE-modified siRNA with a culture medium to achieve a final concentration of 100 nM. As a control, the medium of precultured HeLa cells was replaced with the same amount of culture medium as the above siRNA solution. After 48 hours had elapsed from the medium replacement, the RNA was extracted from each set of cells in the same manner as Example 3, RT-PCR was performed using the obtained total RNA as a template, and the level of expression (amount of mRNA) of the Cyclin B1 gene was measured.

In a similar manner to Example 3, the amount of total RNA extracted from the control cells was used as a reference, and the knock-down efficiency was calculated from the amount of mRNA of the Cyclin B1 gene amplified from the total RNA extracted from the siRNA-introduced cells. The relative value (%) for the level of expression of the Cyclin B1 gene in each of the cells, relative to a value of 100 for the level of expression of the Cyclin B1 gene in the blank (cells prepared by adding only the DMEM medium (low-glucose, 10% FBS, 1% mixed solution of penicillin-streptomycin), is shown in Table 14.

**[Table 14]**

| | Blank | C₅-PFPE |
|---|---|---|
| Relative value (%) | 100 | 52 |

As illustrated in Table 14, in the cells containing the introduced C₅-PFPE-modified siRNA, the level of expression of the Cyclin B1 gene was suppressed approximately 50% compared with the blank. These results confirmed that the Cs-PFPE-modified siRNA had undergone transfection into the target cells, and had functioned normally as an siRNA inside the cells, enabling suppression of expression of the Cyclin B1 gene.

### [Example 11]

The D-aTNA-N[FC10] amidite synthesized in Synthesis Example 4 was linked to an siRNA targeting the luciferase gene to prepare an siRNA with a linked structure (A2).

### <Preparation of SiRNA>

A sense strand (Luc sense) and antisense strand (Luc antisense) comprising the base sequences shown in Table 7 were used for the siRNA for targeting the luciferase gene. The RNA syntheses and identification of each synthesized RNA were conducted in the same manner as Example 5.

An A2-modified sense strand (D-aTNA-N2[FC10]-Luc sense) was synthesized by coupling two molecules of the D-aTNA-N[FC10] amidite to the 5'-terminal of the sense strand.

In a similar manner, another A2-modified sense strand (D-aTNA-N4[FC10]-Luc sense) was synthesized by coupling four molecules of the D-aTNA-N[FC10] amidite to the 5'-terminal of the sense strand. An FAM/A2-modified sense strand (D-aTNA-N4[FC10]-Luc sense-FAM) was also synthesized by introducing fluorescein at the 3'-terminal of this A2-modified sense strand.

Another A2-modified sense strand (Luc sense-D-aTNA-N4[FC10]) was synthesized by coupling four molecules of the D-aTNA-N[FC10] amidite to the 3'-terminal of the sense strand.

Further, a FAM-modified sense strand (Luc sense-FAM) was also synthesized by introducing fluorescein at the 3'-terminal of the sense strand.

Moreover, using a sense strand (GFP sense) and antisense strand (GFP antisense) shown in Table 16, siRNA targeting the GFP gene was obtained. The RNA syntheses and identification of each synthesized RNA were conducted in the same manner as Example 5. The siRNA with no fluorine modification was obtained from a sense strand (GFP sense-T) in which the two uracil bases at the 3'-terminal of the base sequence targeting the GFP gene were substituted with thymidine bases, and an antisense strand (GFP antisense-T) in which the two uracil bases at the 3'-terminal of the base sequence targeting the GFP gene were substituted with thymidine bases. An A2-modified siRNA was obtained from an A2-modified sense strand (D-aTNA-2[FC10]-GFP sense-T) synthesized by coupling two molecules of the D-aTNA-N[FC10] amidite to the 5'-terminal of GFP sense-T, and GFP antisense-T.

**[Table 15]**

| | Base sequence | Mw (M+H+) | |
|---|---|---|---|
| | | Calculated | Found |
| D-aTNA-N2[FC10]-Luc sense | 5'-DD-CUUTACGCUGAGUACUUCGAAAUU-3' | 8549.739 | 8537.864 |
| D-aTNA-N4[FC10]-Luc sense | 5'-DDDD-CUUACGCUGAGUACUUCGAAAUU-3' | 9830.151 | 9821.649 |
| D-aTNA-N4[FC10]-Luc sense-FAM | 5'-DDDD-CUUACGCUGAGUACUUCGAAAUU-FAM-3' | 10403.635 | 10453.929 |
| Luc sense-D-aTNA-N4[FC10] | 5'-CUUACGCUGAGUACUUCGAAAUU-DDDD-3' | 9830.151 | 9820.588 |
| Luc sense-FAM | 5'-CUUACGCUGAGUACUUCGAAAUU-FAM-3' | 7867.887 | 7831.547 |

**[Table 16]**

| | Base sequence | SEQ ID NO: |
|---|---|---|
| GFP antisense-T | 5'-UGCGCUCCUGGACGUAGCCdT*dT-3' | 9 |
| GFP sense-T | 5'-GGCUACGUCCAGGAGCGCAdT*dT-3' | 10 |
| D-aTNA-N2[FC10]-GFP sense-T | 5'-DD-GGCUACGUCCAGGAGCGCAdT*dT-3' | |

In Table 15 and Table 16, "D" means the D-aTNA-N1[FC10] amidite, "FAM" means fluorescein, "*" means a phosphorothioester linkage, and "dT" means a thymidine base.

The unmodified sense strand, the A2-modified sense strand or the FAM/A2-modified sense strand, and the unmodified antisense strand, each of which had been adjusted to a concentration of 8 µM, were denatured in a buffer (0.1 M NaHPO₄, 0.1 M NaH₂PO₄, 1.0 M NaCl, pH 6.99) at 95°C for 10 minutes, and then cooled 5°C every 5 minutes, thus forming a double strand and yielding an siRNA targeting the luciferase gene.

Measurement of the hydrodynamic diameter (nm) of each siRNA was conducted in PBS solution. Measurements were conducted in the same manner as Example 6, using a dynamic light-scattering (DLS) measurement device.

### <Evaluation of Cell Membrane Permeability by Flow Cytometry>

The cell membrane permeability of the siRNA (A2-ssRNA-FAM) containing the FAM/A2-modified sense strand (D-aTNA-N4[FC10]-Luc sense-FAM) was evaluated. The siRNA (ssRNA-FAM) containing the FAM-modified sense strand (Luc sense-FAM) was used for comparison.

First, sample solutions of each siRNA were prepared in the same manner as Example 1. Subsequently, using the same method as Example 1, the medium of HeLa cells that had been precultured at 37°C for 24 hours was replaced with one of the sample solutions, and after culturing at 37°C for 1.5 hours, the collected cells were supplied to flow cytometry and the green fluorescence (488 nm) used for detecting the fluorescein (FAM) was measured. Cells prepared without adding a fluorescein-modified nucleic acid to the medium were used as a blank.

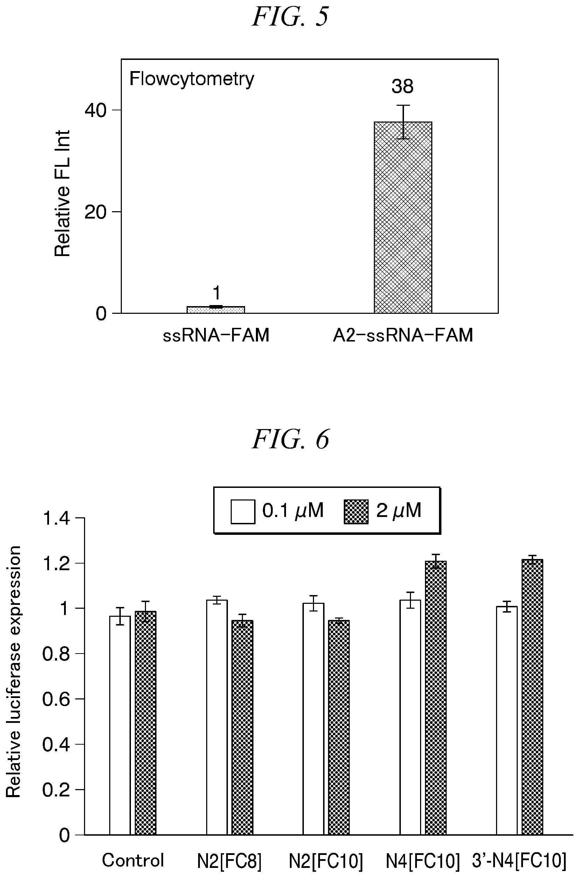

The results of measuring the fluorescent intensity of cells cultured in the mediums containing each siRNA are illustrated in FIG. 5. The fluorescent intensity was calculated as a relative fluorescent intensity relative to a value of 1 for the fluorescent intensity of the cells prepared by adding the control nucleic acid (ssRNA-FAM) to the medium. The results revealed that the fluorescent intensity of the cells prepared by adding the A2-ssRNA-FAM to the medium was dramatically higher than that of the cells prepared by adding the control nucleic acid to the medium, confirming that the A2-ssRNA-FAM had been introduced efficiently into the cells.

### <Confirmation of Energy-Dependent Intracellular Delivery>

The medium of HeLa cells that had been precultured at 37°C for 24 hours was replaced with DMEM liquid medium, and culturing was conducted at 4°C for 0.5 hours. Subsequently, the medium was replaced with a sample solution, and after culturing at either 4°C or 37°C for 1.5 hours, the cell membrane permeability was evaluated. Evaluation of the cell membrane permeability was conducted in the same manner as that described above for the evaluation of cell membrane permeability by flow cytometry.

### <Cell Cultures>

Culturing of HeLa cells was conducted in the same manner as described above with the exception of adjusting the concentration of the mixed solution of penicillin-streptomycin in the culture medium to 1.0%.

### <Knock-Down Assay>

The knock-down activity was investigated for the siRNA containing the A2-Luc sense prepared in Example 5 (the A2-modified sense strand having two molecules of D-aTNA-N[FC8] amidite coupled to the 5'-terminal of the sense strand), and the siRNA containing the D-aTNA-N2[FC10]-Luc sense, the siRNA containing the D-aTNA-N4[FC10]-Luc sense, and the siRNA containing the Luc sense-D-aTNA-N4[FC10] described above in Table 15.

The medium of precultured HeLa cells was replaced with an siRNA solution prepared by diluting the siRNA with a culture medium to achieve a final concentration of either 0.1 µM or 2 µM. As a control, the medium of precultured HeLa cells was replaced with the same amount of culture medium as the above siRNA solution. After 24 hours had elapsed from the medium replacement, a prescribed amount of Luciferase assay reagent was added in accordance with the protocol, and the amount of chemiluminescence was measured using a plate reader. The amount of chemiluminescence is an indicator of the level of luciferase expression.

The results for the relative level of expression of luciferase for each of the cells, relative to a value of 1 for the level of expression of luciferase (the amount of chemiluminescence) for the control cells, are shown in FIG. 6. In FIG. 6, the labels "N2[FC8]", "N2[FC10]", "N4[FC10]" and "3'-N4[FC10]" indicate the cells cultured in solutions containing the siRNA containing the A2-Luc sense, the siRNA containing the D-aTNA-N2[FC10]-Luc sense, the siRNA containing the D-aTNA-N4[FC10]-Luc sense, and the siRNA containing the Luc sense-D-aTNA-N4[FC10] respectively. As illustrated in FIG. 6, no knockdown was observed in any of the cells containing an introduced siRNA.

### <Knock-Down Assay 2>

First, siRNA targeting the GFP gene was introduced into HeLa cells that constantly express GFP (namely, GFP-HeLa cells), and a knock-down assay was then conducted (Rietz et al., Nature Communications, <https://doi.org/10.1038/s41467-020-15300-1>". The siRNA (A2-modified siRNA) formed from the D-aTNA-N2[FC10]-GFP sense-T and the GFP antisense-T described in Table 16 was used as the siRNA. Moreover, siRNA (unmodified siRNA) formed from the GFP sense-T and the GFP antisense-T, and siRNA (cholesterol-modified siRNA) formed from the GFP sense-T and a GFP antisense-T-Chol having cholesterol added at the 3'-terminal of the GFP antisense-T were used as comparative siRNAs.

Specifically, the medium of GFP-HeLa cells that had been precultured at 37°C for 24 hours was replaced with an siRNA solution prepared by diluting each siRNA with a culture medium to achieve a final concentration of either 0.1 µM or 2 µM. After 24 hours had elapsed from the medium replacement, the amount of GFP fluorescence was measured using a plate reader. This amount of fluorescence is an indicator of the level of GFP expression. Further, quantification of the mRNA associated with the GFP expression was conducted by q-PCR.

### <Knock-Down Assay in Presence of Chloroquine>

Chloroquine, which is a compound known to destroy endosomes, was added, and a knock-down assay was then conducted. Specifically, the medium of HeLa cells that had been precultured at 37°C for 24 hours was replaced with an siRNA solution prepared by diluting the siRNA with a culture medium to achieve a final concentration of either 0.1 µM or 2 µM, and culturing was then conducted at 37°C for 6 hours. Subsequently, a 100 µM chloroquine solution was added to the siRNA solution, and after conducting culturing at 37°C for 18 hours, the level of luciferase expression was evaluated.

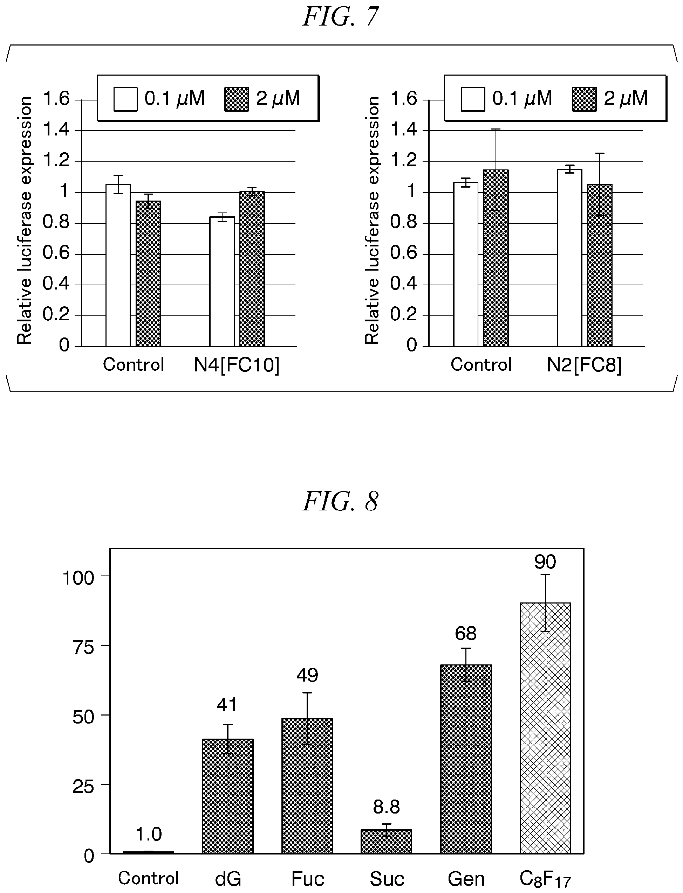

The results for the relative level of expression of luciferase for each of the cells, relative to a value of 1 for the level of expression of luciferase (the amount of chemiluminescence) for the control cells, are shown in FIG. 7. In FIG. 7, the labels "N2[FC8]" and "N4[FC10]" have the same meaning as in FIG. 6. As illustrated in FIG. 7, no knockdown was observed in either the siRNA modified with the D-aTNA-N4[FC10] or the siRNA modified with D-aTNA-N2[FC8].

### <Knock-Down Efficiency when Transfection Reagent Used>

The medium of HeLa cells that had been precultured at 37°C for 24 hours was replaced with a DMEM liquid medium containing a mixed solution of a transfection reagent (lipofectamine) and a sample (siRNA), and following culturing at 37°C for 1.5 hours, the cell membrane permeability was evaluated. Evaluation of the cell membrane permeability was conducted in the same manner as that described above for the evaluation of cell membrane permeability by flow cytometry.

### [Example 12]

Using the same procedure as Example 6, the cellular uptake efficiency of fluorescein-modified nucleic acids having one of the structures (A1) to (A4), and a fluorescein-modified nucleic acid having none of the structures (A1) to (A4) was investigated by quantitative analysis using flow cytometry. The fluorescein-modified nucleic acids having one of the structures (A1) to (A4) were synthesized in the same manner as Example 6, using the phosphoramidites having cell membrane permeability synthesized in the various synthesis examples described above (D-aTNA-N[C8], L-aTNA-N[FC8], D-aTNA-N[FC8], D-aTNA-N[FC10], and D-aTNA-N[C12]).

### <Evaluations of Physical Properties of Fluorescein-Modified Nucleic Acids>

For each of the fluorescein-modified nucleic acids, the hydrodynamic diameter (nm) in a PBS solution, the zeta potential (mV) in a 0.2× PBS solution, and the critical micelle concentration (CMC) (µM) in a PBS solution were measured. The hydrodynamic diameter (nm) and the critical micelle concentration (CMC) (µM) were measured using the same methods as described in Example 6.

The zeta potential (mV) of each fluorescein-modified nucleic acid in a 0.2× PBS solution was measured by dissolving a sample of the fluorescein-modified nucleic acid in a 0.2× PBS solution to form a test sample solution of concentration 10 µM, and then conducting a measurement using a DLS measurement device. The measurement with the DLS measurement device was conducted in the same manner as Example 6.

The results of measuring the zeta potential (mV) of each fluorescein-modified nucleic acid in a 0.2× PBS solution are shown in Table 17.

**[Table 17]**

| Fluorescein-modified nucleic acid | Zeta potential [mV] |
|---|---|
| Control | -33±2 |
| D-aTNA-N2[C8] | -42±3 |
| D-aTNA-midN2[FC8] | -45±1 |
| L-aTNA-N2[FC8] | -42±3 |
| D-aTNA-N2[FC8] | -50±2 |
| D-aTNA-N2[C12] | -48±3 |
| D-aTNA-N2[FC10] | -53±2 |

### <Evaluation of Cell Membrane Permeability in Presence of Inhibitor>

The medium of HeLa cells that had been precultured at 37°C for 24 hours was replaced with a DMEM liquid medium containing an inhibitor, and culturing was then conducted at 37°C for one hour. Subsequently, the medium was replaced with a DMEM solution containing D-aTNA-N2[FC10], and following culturing at 37°C for 1.5 hours, the cell membrane permeability was evaluated. Evaluation of the cell membrane permeability was conducted in the same manner as that described above for the cell membrane permeability evaluations performed by flow cytometry. The inhibitors used were as follows: deoxyglucose (dG, 50 mM) as an endocytosis inhibitor, fucoidan (Fuc, 0.5 mg/L) as a scavenger receptor inhibitor, sucrose (Suc, 0.3 mM) as a clathrin-mediated endocytosis inhibitor, and genistein (Gen, 700µM) as a caveolae-mediated endocytosis inhibitor.

The fluorescent intensity was measured for each of the cells, and a relative fluorescent intensity was determined relative to a value of 1.0 for cells that had been cultured at 37°C with only the control nucleic acid added to the medium. The results are shown in FIG. 8. In the presence of sucrose, deoxyglucose or fucoidan, the relative fluorescent intensity of cells containing the fluorescein-modified nucleic acid in which R¹⁰⁰ represents a perfluoroalkyl group decreased more than the cells containing no added inhibitor (shown as "C₈F₁₇" in the figure). These results suggested that cellular uptake of the D-aTNA-N2[FC10] is due to clathrin-mediated endocytosis. Further, the results also suggested that intracellular delivery was dependent on the scavenger receptor inhibitor.

### <Measurement of DNA Density per Particle>

The weight average molecular weight (Mw) of each fluorescein-modified nucleic acid was measured by dissolving a sample of the fluorescein-modified nucleic acid in PBS to form a test sample solution of concentration 2 µM, and then measuring the test sample using a static light-scattering (SLS) measurement device. The DNA density per particle is then calculated by dividing the apparent molecular weight per particle obtained from the SLS measurement by the molecular weight per molecule. SLS is a technique for measuring the absolute molecular weight according to Rayleigh theory, based on the relationship between the intensity of light scattered by the molecules, and the molecular weight and size of the molecules. Measurements were conducted using the same equipment as that used for measurement of the hydrodynamic diameter (nm) of each fluorescein-modified nucleic acid in Example 6, with the scattered light measured at different DNA densities. The same sample was measured three or more times, and the weight average molecular weight was calculated from those results using a Debye plot.

**[Table 18]**

| Fluorescein-modified nucleic acid | DNA density per particle |
|---|---|
| D-aTNA-N2[C6] | 149±19 |
| D-aTNA-N2[FC8] | 561±33 |
| D-aTNA-N2[FC10] | 208±17 |

### <Measurement of Fluorine Dispersion within Particles>

Small-angle X-ray scattering (SAXS) is an analytical technique for evaluating the structure of a substance by measuring those X-rays scattered upon irradiation of the substance that are observed in the low-angle region of 2θ < 10°, and can be used to evaluate the shape and electron density of microparticles. By using this technique to investigate the state of the electron density of particles formed by the fluorine-modified nucleic acid in water, the presence or absence of core-shell structure formation, and the distribution of fluorine atoms within the micelles can be clarified.

### [Industrial Applicability]

The present invention is able to provide a nucleic acid for transfection that has favorable cell membrane permeability attributable to a perfluoroalkyl group, and also provides a method for producing the nucleic acid for transfection. Because the nucleic acid for transfection according to the present invention exhibits excellent cell membrane permeability, and therefore enables the introduction of even siRNA into a cell without requiring the use of a transfection reagent, it holds much promise for application in the fields of medicine and biotechnology.

## Claims

1. A nucleic acid for transfection in which a nucleic acid targeted for introduction into a cell and a cell membrane-permeable group are linked together, and
the cell membrane-permeable group has a structure represented by one of general formulas (A1) to (A4) shown below: 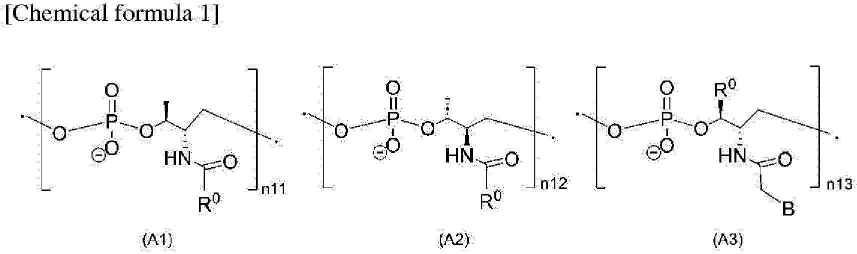 (wherein in formulas (A1) to (A3), R⁰ represents an alkyl group of 1 to 30 carbon atoms substituted with one or more fluorine atoms, a group composed of an alkyl group of 2 to 30 carbon atoms substituted with one or more fluorine atoms and having one to five ether-bonded oxygen atoms between carbon atoms, an alkyl group of 10 to 30 carbon atoms not substituted with any fluorine atoms, or a group composed of an alkyl group of 10 to 30 carbon atoms not substituted with any fluorine atoms but having one to five ether-bonded oxygen atoms between carbon atoms; n11, n12 and n13 each independently represent an integer of 1 or greater; B represents a nucleic acid base; and black dots indicate bonding sites) 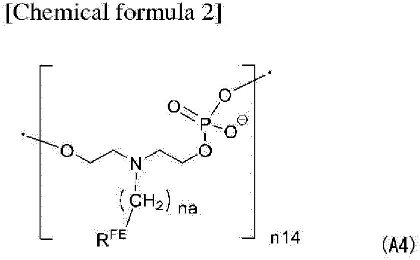 (wherein in formula (A4), R^{FE} represents a perfluoroalkyl group of 1 to 10 carbon atoms having no ether-bonded oxygen atoms between carbon atoms, or a perfluoroalkyl group of 2 to 10 carbon atoms having one to five ether-bonded oxygen atoms between carbon atoms; na represents an integer of 1 to 10; n14 represents an integer of 1 or greater; and black dots represent bonding sites).

2. The nucleic acid for transfection according to Claim 1, wherein the nucleic acid targeted for introduction into a cell is an siRNA.

3. The nucleic acid for transfection according to Claim 1 or 2, wherein R⁰ represents an alkyl group of 1 to 30 carbon atoms substituted with at least two fluorine atoms.

4. The nucleic acid for transfection according to Claim 1 or 2, wherein R⁰ represents a perfluoroalkyl group of 1 to 10 carbon atoms, or a group composed of a perfluoroalkyl group of 1 to 10 carbon atoms having one to five ether-bonded oxygen atoms between carbon atoms.

5. The nucleic acid for transfection according to Claim 1 or 2, wherein R⁰ represents an alkyl group of 10 to 30 carbon atoms not substituted with any fluorine atoms, or a group composed of an alkyl group of 10 to 30 carbon atoms not substituted with any fluorine atoms but having one to five ether-bonded oxygen atoms between carbon atoms.

6. The nucleic acid for transfection according to Claim 1 or 2, wherein n11 is 2 or greater, n12 is 2 or greater, n13 is 2 or greater, and n14 is 2 or greater.

7. A nucleic acid transfection method, the method comprising bringing the nucleic acid for transfection according to Claim 1 or 2 into contact with a cell to introduce the nucleic acid into the cell.

8. A method for producing a cell membrane permeability-improved siRNA having a cell membrane-permeable group linked to an siRNA, wherein
the cell membrane-permeable group has a structure represented by general formula (A1), (A2) or (A3) shown below: 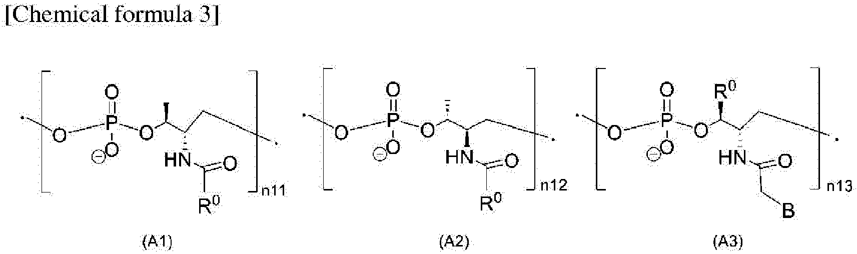 (wherein in formulas (A1) to (A3), R⁰ represents an alkyl group of 1 to 30 carbon atoms substituted with one or more fluorine atoms, a group composed of an alkyl group of 2 to 30 carbon atoms substituted with one or more fluorine atoms and having one to five ether-bonded oxygen atoms between carbon atoms, an alkyl group of 10 to 30 carbon atoms not substituted with any fluorine atoms, or a group composed of an alkyl group of 10 to 30 carbon atoms not substituted with any fluorine atoms but having one to five ether-bonded oxygen atoms between carbon atoms; n11, n12 and n13 each independently represent an integer of 1 or greater; B represents a nucleic acid base; and black dots indicate bonding sites), and
the method comprises a step of linking the cell membrane-permeable group either directly or indirectly to a sense strand of the siRNA by a phosphoramidite method using a phosphoramidite having the cell membrane-permeable group as a raw material.

9. An anticancer agent comprising, as an active ingredient, a molecule in which an siRNA that targets a cancer gene and a cell membrane-permeable group are linked together either directly or indirectly, and
the cell membrane-permeable group has a structure represented by one of general formulas (A1) to (A4) shown below: 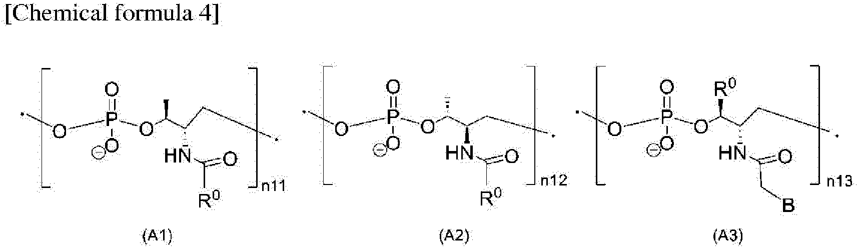 (wherein in formulas (A1) to (A3), R⁰ represents an alkyl group of 1 to 30 carbon atoms substituted with one or more fluorine atoms, a group composed of an alkyl group of 2 to 30 carbon atoms substituted with one or more fluorine atoms and having one to five ether-bonded oxygen atoms between carbon atoms, an alkyl group of 10 to 30 carbon atoms not substituted with any fluorine atoms, or a group composed of an alkyl group of 10 to 30 carbon atoms not substituted with any fluorine atoms but having one to five ether-bonded oxygen atoms between carbon atoms; n11, n12 and n13 each independently represent an integer of 1 or greater; B represents a nucleic acid base; and black dots indicate bonding sites) 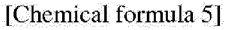 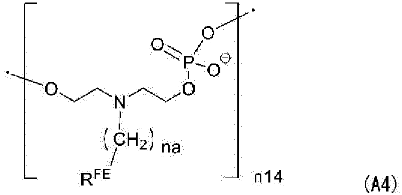 (wherein in formula (A4), R^{FE} represents a perfluoroalkyl group of 1 to 10 carbon atoms having no ether-bonded oxygen atoms between carbon atoms, or a perfluoroalkyl group of 2 to 10 carbon atoms having one to five ether-bonded oxygen atoms between carbon atoms; na represents an integer of 1 to 10; n14 represents an integer of 1 or greater; and black dots represent bonding sites).
